# EUROPEAN PATENT SPECIFICATION

(11) **EP 4 319 565 B1**
(45) Date of publication and mention of the grant of the patent: **19.11.2025**
(21) Application number: 22821248.6
(22) Date of filing: 08.06.2022
(51) Int. Cl.: A23L 13/00, A23L 13/40, A23P 10/40, C12N 5/071, C12N 5/00

(54) **COMESTIBLE CELL-BASED MEAT PRODUCTS COMPRISING DRY CELL POWDER AND METHODS OF MAKING SUCH PRODUCTS**
ESSBARE ZELLBASIERTE FLEISCHPRODUKTE MIT TROCKENZELLPULVER UND VERFAHREN ZUR HERSTELLUNG SOLCHER PRODUKTE
PRODUITS CARNÉS À BASE DE CELLULES COMESTIBLES COMPRENANT UNE POUDRE DE CELLULES SÈCHES ET PROCÉDÉS DE FABRICATION DE TELS PRODUITS

(30) Priority: 11.06.2021 US 202163202466 P
(43) Date of publication of application: 14.02.2024
(60) Divisional application: 25204933.3
(73) Proprietor: Upside Foods, Inc., Berkeley CA 94710 (US)
(72) Inventor: REASE, Morgan, Laurence, Berkeley, CA 94710 (US); WICKE, Antony, Berkeley, CA 94710 (US)
(74) Representative: Meissner Bolte Nürnberg
(86) International application number: PCT/US2022/072825
(87) International publication number: WO 2022/261647

(56) References cited:
- WO-A1-2015/120174
- WO-A1-2018/189738
- WO-A1-2019/016795
- WO-A1-2020/100143
- WO-A1-2022/047263
- CN-A- 111 513 263
- US-A1- 2021 037 870

## Description

### BACKGROUND

As the world's population continues to grow, the need for meat products for consumption is greater than ever. Unfortunately, producers of conventional slaughtered meat products are struggling to meet the demand. Cell-based or cultured meat products for consumption have emerged as an attractive alternative (or supplement) to conventional slaughtered meat from animals. For instance, cell-based, cultured, or cultured meat represents a technology that could address the specific dietary needs of humans. Cell-based meat products can be prepared from cultured cells derived from a non-human animal. Because the cells for cell-based meat are made in a food cultivation facility, cell masses are often formed and shaped to mimic textures and forms of conventional meat.

In addition to addressing dietary needs, cell-based-meat products help alleviate several drawbacks linked to conventional slaughtered meat products. For instance, conventional meat production involves controversial practices associated with animal husbandry and slaughter. Other drawbacks associated with slaughtered meat production include low conversion of caloric input to edible nutrients, microbial contamination of the product, emergence and propagation of veterinary and zoonotic diseases, relative natural resource requirements, and resultant industrial pollutants, such as greenhouse gas emissions and nitrogen waste streams. WO2020100143 and US2021-0037870 describe the use of cultured animal cells in a meat substitute, in WO2019016795 animal cells are grown on a scaffold to mimic meat, CN111513263 describes the addition of transglutaminase and casein to animal cell cultures to produce a meat substitute, WO2018189738 describes the combination of animal cell cultures with plant-derived substances to form a meat substitute, in WO2015/120174 dried animal cell cultures are combined with plant-derived hydrogels to form an edible product, and in WO2022/047263 dried animal cell cultures are used in preparing a food composition.

Despite advances in creating cell-based-meat products, existing methods for cultivating and processing cell-based meat products face several challenges, such as mimicking the textures and flavors of slaughtered meat to meet consumer expectations and preferences. In particular, existing methods can produce cell-based-meat products with less-than-optimal textures and flavors. Indeed, after being harvested from a bioreactor, cultured cell-based-meat products typically comprise a wet, soft, and malleable consistency and texture that is distinct from the texture and consistency of slaughtered meat. Along similar lines, some cultured cell-based-meat products fall short of mimicking the taste of slaughtered meat, largely due to the differences in texture and consistency. These along with additional problems and issues exist with regard to preparation of cell-based meat products.

The subject matter claimed herein is not limited to embodiments that solve any disadvantages or that operate only in environments such as those described above. Rather, this background is only provided to illustrate one example technology area where some embodiments described herein may be practiced.

### BRIEF SUMMARY

This disclosure generally describes comestible cell-based meat products including a combination of wet cultured cells and dry cell powder and methods for preparing such cell-based meat products. In one or more embodiments, the comestible cell-based meat product comprises a mixture of wet cultured animal cells and dry cultured animal cell powder. For example, the comestible cell-based meat product is produced by generating a homogenous mixture of wet cultured animal cells and dry cultured animal cell powder. In one or more embodiments, the dry cultured animal cell powder improves the texture of the resultant comestible cell-based meat product by tailoring the hardness and/or adhesiveness of the comestible cell-based meat product to be more consistent with that of slaughtered meat.

Additional features and advantages of one or more embodiments of the present disclosure are outlined in the description which follows, and in part will be obvious from the description, or may be learned by the practice of such example embodiments.

### BRIEF DESCRIPTION OF THE DRAWINGS

The detailed description provides one or more embodiments with additional specificity and detail through the use of the accompanying drawings, as briefly described below.
FIG. 1 illustrates a flowchart for forming a comestible cell-based food product utilizing cultured animal cells and dry cultured animal cell powder in accordance with one or more embodiments.
FIG. 2 illustrates an example process for preparing a homogenous mixture of adherent cells and dry cultured animal cell powder in accordance with one or more embodiments.
FIG. 3 illustrates an example process for preparing adherent cells in accordance with one or more embodiments.
FIG. 4 illustrates an example process for preparing suspension cells in accordance with one or more embodiments.
FIG. 5 is a chart showing hardness at 60% strain compression of raw and cooked comestible cell-based food products with various proportions of dry cultured animal cell powder in accordance with one or more embodiments.
FIG. 6 is a chart showing adhesiveness at 60% strain compression of raw and cooked comestible cell-based food products with various proportions of dry cultured animal cell powder in accordance with one or more embodiments.
FIG. 7 illustrates hardness at 90% strain compression of raw comestible cell-based food products with various proportions of dry cultured animal cell powder in accordance with one or more embodiments.
FIG. 8 illustrates adhesiveness at 90% strain compression of raw comestible cell-based food products with various proportions of dry cultured animal cell powder in accordance with one or more embodiments.
FIG. 9 illustrates a flowchart of a series of acts for preparing a comestible cell-based food product utilizing dry cultured animal cell powder in accordance with one or more embodiments.
FIG. 10 illustrates a flowchart of a series of acts for tailoring one or more texture properties of a comestible cell-based food product utilizing dry cultured animal cell powder in accordance with one or more embodiments.

### DETAILED DESCRIPTION

This disclosure describes one or more embodiments of a method for tailoring one or more properties of a comestible cell-based food product utilizing dry cultured cell powder. To illustrate, the comestible cell-based food product generally contain cultured animal tissues and dry cultured animal cell powder. The dry cultured animal cell powder can modify the texture of the cultured animal tissues, which in turn can enhance the taste of the comestible cell-based food product. For example, the dry cultured animal cell powder can modify the hardness, adhesiveness, and/or other properties of the cultured animal cell-based tissues to tailor the texture of the resultant comestible cell-based food product.

In one or more embodiments, the cultured animal tissues are prepared from adherent and/or suspension cells derived from a non-human animal. Further, in one or more embodiments, the comestible cell-based food product is prepared by combining the adherent cells and/or the suspension cells with dry cultured animal cell powder. By way of example, the comestible cell-based food product may be a meat product produced from adherent cells, suspension cells, and/or dry cultured animal cell powder prepared using cells taken from one or more animals including, but not limited to, cows, pigs, ducks, chickens, and fish.

As mentioned above, the comestible cell-based food product can comprise cultured animal cells and dry cultured animal cell powder. More specifically, in one or more embodiments, the comestible cell-based food product contains cultured adherent cells and dry cultured animal cell powder. In addition, or in the alternative, the comestible cell-based food product includes cultured suspension cells and dry cultured animal cell powder. Further, in some embodiments, the comestible cell-based food product includes a combination of cultured adherent cells, cultured suspension cells, and dry cultured animal cell powder.

As mentioned above, the comestible cell-based food product can comprise dry cultured animal cell powder. In some embodiments, the dry cultured animal cell powder is prepared by spray drying cultured suspension cells to form a powder. Alternatively, in one or more embodiments, the dry cultured animal cell powder utilized to form the comestible cell-based food product is formed by dehydrating cultured adherent tissue and grinding the dried cultured adherent tissue into a powder. Dry cultured animal cell powder enhances the comestible cell-based food product by providing additional flavor and texture. Comestible cell-based food product produced with dry cultured animal cell powder can better mimic the natural flavor and texture of conventional slaughtered meat products.

One or more implementations involve tailoring one or more properties of a comestible cell-based food product utilizing dry cultured animal cell powder. More specifically, one or more implementations involve tailoring one or more properties that affect the texture of a comestible cell-based food product by controlling an amount of dry cultured animal cell powder in the comestible cell-based food product. For example, one or more implementations involve controlling an amount of dry cultured animal cell powder in the comestible cell-based food product to modify or tailor one or more of the hardness, adhesiveness, resilience, cohesion, springiness, gumminess, or chewiness of the comestible cell-based food product.

One or more implementations involve controlling the amount of dry cultured animal cell powder in a comestible cell-based food product based on the type of cultured animal cells used in the comestible cell-based food product. In particular, the amount of dry cultured animal cell powder that will achieve a desired texture can vary based on the type of cells used to create the comestible cell-based food product. For example, an adherent cell-based food product may require less dry cultured animal cell powder than a suspension cell-based food product to achieve a desired texture. Along similar lines, a comestible cell-based food product comprising both adherent cells and suspension cells may require more dry cultured animal cell powder than a suspension cell-based food product and less dry cultured animal cell powder than an adherent cell-based food product.

Furthermore, one or more implementations involve controlling the amount of dry cultured animal cell powder in a comestible cell-based food product based on the type of comestible cell-based food product. In particular, a target hardness for a comestible cell-based steak product may be higher than a target hardness for a comestible cell-based ground beef product. Thus, one or more implementations involve including more amount of dry cultured animal cell powder in a comestible cell-based steak product than a comestible cell-based ground beef product. Similarly, a target hardness for a comestible cell-based chicken product may be higher than a target hardness for a comestible cell-based shrimp or lobster product.

The disclosed comestible cell-based food products provide one or more advantages and benefits. More specifically, the disclosed comestible cell-based food product includes improved texture and flavor relative to existing methods or systems. By preparing the comestible cell-based food product utilizing dry cultured animal cell powder, the comestible cell-based food product can include improved hardness and adhesion, which results in a texture more consistent with traditional meat products (e.g., slaughtered meat). Further, the disclosed methods allow flexibility in controlling the texture of comestible cell-based food products based on the type of cells or type of food product.

Additionally, the comestible cell-based food products described herein have a number of unique features and advantages compared to conventional, slaughtered meat products. The cultivation methods described herein can be tailored to achieve desired traits such as health and sensory benefits. For example, as compared to conventional products, the comestible cell-based food products of the disclosure comprise a significantly lower amount of steroid hormones. For example, using the culturing methods described, there need not be any exogenous hormones added into culture thus resulting in lower or non-existent hormonal levels in a resulting cell-based meat product. Accordingly, in some embodiments, the comestible cell-based food product is substantially free of steroid hormones (i.e., contains little or no steroid hormones). This offers an improvement over conventional, slaughtered meat products, which often include various exogenous hormones fed or otherwise administered to animals before slaughter.

Accordingly, in some embodiments, the comestible cell-based food product of the disclosure comprises no more than about 1ug, 0.5ug, 0.1ug, 0.05ug, 0.01ug, 0.005ug, or even about 0.001ug steroid hormone/kg dry mass of comestible cell-based food product. Additionally, in one or more embodiments, the comestible cell-based food product comprises no more than about 1ug, 0.5ug, 0.1ug, 0.05ug, 0.01ug, 0.005ug, or even about 0.001ug progesterone/kg dry mass of comestible cell-based food product. Further, in some embodiments, the comestible cell-based food product comprises no more than about 1ug, 0.5ug, 0.1ug, 0.05ug, 0.01ug, 0.005ug, or even about 0.001ug testosterone/kg dry mass of comestible cell-based food product. In some embodiments, the comestible cell-based food product comprises no more than about 0.05ug, 0.01ug, 0.005ug, or even about 0.001ug estradiol/kg dry mass of comestible cell-based food product. In exemplary embodiments, the comestible cell-based food product comprises no more than about 35 ng estradiol/kg dry mass of comestible cell-based food product.

Additionally, the disclosed methods improve sterility of the produced comestible cell-based food product by utilizing sterile, laboratory-based cell culturing methods. Thus, the comestible cell-based food product is substantially free of microbial contaminants. "Substantially free" means that the concentration of microbes or parasites is below a clinically significant level of contamination, i.e., below a level wherein ingestion would lead to disease or adverse health conditions. Such low levels of contamination allow for an increased shelf life. This is in contrast to animals raised for conventional meat production. As used herein, microbial contamination includes, but is not limited to, bacteria, fungi, viruses, prions, protozoa, and combinations thereof. Harmful microbes may include coliforms (fecal bacteria), *E. coli,* yeast, mold, *Campylobacter, Salmonella, Listeria,* and Staph. In addition, cells grown in culture may be substantially free from parasites such as tapeworms that infect cells of whole animals and that are transferred to humans through consumption of insufficiently cooked meat.

Further, relative to conventional products, the present comestible cell-based food product reduces inclusion of antibiotics. Comestible cell-based food products of the disclosure comprise a significantly lower amount of antibiotics, or are substantially free of antibiotics, or are free of antibiotics entirely. For example, using the cell culturing methods described herein, the use of antibiotics in culture can be controlled or eliminated, thus resulting in lower or non-existent antibiotic levels in the resulting comestible cell-based food product. Accordingly, in some embodiments, the comestible cell-based food product is substantially free of antibiotics (i.e., contains little or no antibiotics). This is in contrast to animals raised for conventional meat production, which are often fed or otherwise administered exogenous antibiotics.

Accordingly, in some embodiments, the comestible cell-based food product of the disclosure comprises no more than about 100 ug antibiotics/kg dry mass of comestible cell-based food product, 90 ug antibiotics/kg dry mass of comestible cell-based food product, 80 ug antibiotics/kg dry mass of comestible cell-based food product, 70 ug antibiotics/kg dry mass of comestible cell-based food product, 60 ug antibiotics/kg dry mass of comestible cell-based food product, 50 ug antibiotics/kg dry mass of comestible cell-based food product, 40 ug antibiotics/kg dry mass of comestible cell-based food product, 30 ug antibiotics/kg dry mass of comestible cell-based food product, 20 ug antibiotics/kg dry mass of comestible cell-based food product, 10 ug antibiotics/kg dry mass of comestible cell-based food product, 5 ug antibiotics/kg dry mass of comestible cell-based food product, 1 ug antibiotics/kg dry mass of comestible cell-based food product, 0.5 ug antibiotics/kg dry mass of comestible cell-based food product, 0.1 ug antibiotics/kg dry mass of comestible cell-based food product, 0.05 ug antibiotics/kg dry mass of comestible cell-based food product, or even about 0.01 ug/kg of antibiotics/kg dry mass of comestible cell-based food product.

Additionally, compared to conventional products, the comestible cell-based food products of the disclosure comprise a lower average total lipid (fat) content. For example, cell-based meat generally has an average total fat content between about 0.5% to about 10.0%, whereas the fatty acid content in conventional meat varies widely and can range from about 3% to about 18%, depending on the cut of meat.

Accordingly, in some embodiments, the comestible cell-based food product of the disclosure comprises an average total fat content of about 0.5%-10% , when measured as a % of total wet mass of the comestible cell-based food product. A lower fat content provides a lower caloric content, as well as other related health benefits, when compared to conventional products. The methods provided herein can alter specific fatty acid profiles to achieve desired flavor characteristics or fatty acid profiles. The lower levels of fatty acids in the cell-based product of the disclosure also promotes an increased shelf life, for example by leading to lower levels of fatty oxidation in the product.

Additionally, the disclosed comestible cell-based food product improves shelf life over conventional slaughtered meat products. A significant portion of meat and meat products are spoiled every year. It is estimated that approximately 3.5 billion kg of poultry and meat are wasted at the consumer, retailer and foodservice levels which have a substantial economic and environmental impact. A significant portion of this loss is due to microbial spoilage.

Conventional meat is perishable and has a relatively short shelf life stability (interchangeably referred to as simply "shelf life" herein). The shelf life is the amount of time a food remains fit for human consumption. The composition of conventional meat and the conditions used to slaughter and harvest the meat create favorable growth conditions for various microorganisms including fecal bacteria (e.g., coliform bacteria). Conventional meat products are also very susceptible to spoilage due to chemical, oxidative and enzymatic activities. It is generally regarded that microbial growth, oxidation and enzymatic autolysis are three mechanisms responsible for the spoilage of meat. The breakdown of fat, protein, and carbohydrates of meat results in the development of off-odors and off-flavor and these the off-odors and off-flavors make the meat objectionable for human consumption. Depending on the species and method of harvest, conventional meat products are not safe to consume after a relatively short period of storage time. For example, chicken should be cooked within a few days of purchasing. Cooked poultry can be safely stored in the fridge for only 4 days and the freezer for up to 4 months. It is, therefore, necessary to control meat spoilage in order to increase its shelf life and maintain its nutritional value, texture, and flavor.

Cultured cell-based meat, including the disclosed comestible cell-based food product, through its method of production and composition, produces a meat product that has extended shelf life compared to conventional meat products. To illustrate, the disclosed comestible cell-based food product does not require the addition of preservative agents to obtain the shelf-life stability. In addition, the manufacturing methods used to produce cell-based meat require clean and aseptic conditions. These conditions ensure that microbial cell counts in both harvested products and subsequent food processing are low. These multiple factors contribute to extended shelf-life stability of cell-based meat.

The shelf life due to spoilage of the disclosed comestible cell-based food product is enhanced relative to conventional meat. This is the case both at room temperature (about 25°C) and at colder temperatures (e.g., about 4°C). The increased shelf life is associated with reduced contamination, composition of the cell-based meat, reduced degradation of the cell-based meat and slower rates of change in color, spoilage, smell, and flavor of the cell-based meat.

The comestible cell-based food products of the disclosure can include a higher Vitamin E (αTocopherol) content over conventional, slaughtered meat products. In some embodiments, the cell-based product of the disclosure comprises at least about 0.5mg, at least about 0.6mg, at least about 0.7mg, at least about 0.8mg, at least about 0.9mg, or at least about 1.0mg/Vitamin E/100g wet mass of cell-based product.

Additionally, by way of example, the comestible cell-based food products, unless otherwise manipulated to include, do not include vascular tissues, such as veins and arteries, whereas conventional meat does contain such vasculature, and contains the blood found in the vasculature. Accordingly, in some embodiments, the comestible cell-based food products does not comprise any vasculature.

As illustrated by the foregoing discussion, the present disclosure utilizes a variety of terms to describe features and advantages of the disclosed methods. Additional detail is now provided regarding the meaning of such terms. For example, as used herein, the term "cells" refers to individual cells of meat. In particular, cells may comprise different cell types, such as one or more of muscle-derived cells, muscle progenitor cells, satellite cells, stem cells, myoblasts, mesangioblasts, myofibroblasts, mesenchymal stem cells, hepatocytes, fibroblasts, pericytes, adipocytes, epithelial, chondrocytes, osteoblasts, osteoclasts, pluripotent cells, somatic stem cells, endothelial cells, or other similar cell types. Furthermore, cells may comprise different types of progenitor cells, including myogenic progenitors, adipogenic progenitors, mesenchymal progenitors, or other types of progenitor cells.

As used herein, the term "suspension cells" (or "suspension") refers to cells growing in an at least partially liquid growth medium in which cells grow, multiply, and/or maintain nourishment. In particular, a suspension includes an agitated growth medium that is housed in a container in which single cells or small aggregates of cells grow, multiply, and/or maintain nourishment from the nutrients of the agitated growth medium. Cells grown in suspension are not attached to a substrate and therefore differ from a conventional adherent culture. As used herein, the term "suspension culture" or "cell suspension culture" refers to a type of culture in which single cells or small aggregates of cells are cultured as non-adherent cells or aggregates of cells.

As used herein, the term "adherent cells" refers to a mass comprising cells of meat. In particular, adherent cells can refer to cellular tissue of cultured meat gathered into a collective mass, including via growth on a substrate. In some embodiments, the cell mass is comestible. Additionally, adherent cells can include cells grown on a substrate that have been nourished by a growth medium to grow during a formation period. Adherent cells may comprise different cell types, such as one or more of muscle-derived cells, muscle progenitor cells, satellite cells, stem cells, myoblasts, mesangioblasts, myofibroblasts, mesenchymal stem cells, hepatocytes, fibroblasts, pericytes, adipocytes, epithelial, chondrocytes, osteoblasts, osteoclasts, pluripotent cells, somatic stem cells, endothelial cells, or other similar cell types. For example, adherent cells can include a cell sheet of cultured meat growing within an enclosure, such as a chamber, housing, container, etc.

As used herein, the phrases "cell-based meat composition," "cell-based meat," "slaughter-free meat," "slaughter-free cell-based meat," "in vitro produced meat," " in vitro cell-based meat," "cultured meat," "slaughter-free cultured meat," "in vitro produced cultured meat," "in vitro meat," "in vitro cultured meat" and other similar such phrases are interchangeably used herein, and refer to the meat that is generated in vitro, starting with cells in culture, and that method which does not involve the slaughter of an animal in order to directly obtain meat from that animal for dietary consumption.

As used herein, the term "substrate" refers to a material on which cells attach or grow. In particular, a substrate includes a material to which cells adhere and upon which cells form a cellular tissue. Accordingly, a substrate can support or promote cell adhesion, cell differentiation, and/or growth of cells to form a cell mass-namely, a comestible meat product. For example, a steel substrate or other substrate can be positioned to receive loaded cell culture media as part of a seeding process inside a bioreactor. Once the cell mass grows to a predetermined size or for a predetermined duration, in some embodiments, the cell mass is harvested from the substrate. The substrate can include a variety of bio-compatible materials, such as a metal material, polymer material, organic, or biologic scaffold.

Additionally, as used herein, the term "dry cultured animal cell powder" refers to dried cultured non-human animal cells. In particular, dry cultured animal cell powder can include spray-dried suspension cells. Further, in one or more embodiments, dry cultured animal cell powder can include adherent cells that have been dried and ground, crushed, or pulverized. Similar to the discussion above with regard to suspension cells and adherent cells, dry cultured animal cell powder can include a variety of different cell types.

As used herein, the term "texture" refers to mechanical characteristics of a cell-based meat composition that are correlated with sensory perceptions of the cell-based meat composition. As used herein, the terms "texture profile analysis" or "TPA" refers to the double compression test or similar test for determining the textural properties of a cell-based meat composition. A TPA test uses multiple rounds of compression as a texture analyzer to provide insight into how samples behave when chewed. In some cases, TPA quantifies hardness, cohesiveness, springiness, and resilience.

As used herein, the terms "adhesive capability" or "adhesiveness" refer to a TPA parameter that quantifies a material's tendency to adhere to a probe. As used herein, the term "chewiness" refers to a TPA parameter that is calculated as the product of the TPA parameters gumminess and springiness. Without wishing to be bound by theory, chewiness is thought to express the energy required to chew a food product to a state where it is ready for swallowing. Non-limiting variables that can be titrated to modulate the chewiness of the meat-like food products provided herein include but are not limited to densities of textured proteins, and moisture content.

As used herein, the term "cohesiveness" refers to a TPA parameter that is calculated from the area of work during the first compression of the cell-based meat composition. Without wishing to be bound by theory, cohesiveness is thought to express the structural integrity of a food product, and refers to a property characterized by the strength of internal bonding that makes up the body of a cell based meat composition. Non-limiting variables that can be titrated to modulate the cohesiveness of the meat-like cell based meat composition provided herein include but are not limited to types and amounts of binding agents.

As used herein, the term "hardness" refers to a texture parameter of a food product and is calculated from the peak force of the first compression of the cell-based meat composition in either a TPA assay or a compression assay. Without wishing to be bound by theory, "hardness" is thought to correlate with the force required to compress a cell based meat composition between molars during chewing. Non-limiting variables that can be titrated to modulate the hardness of the cell based meat compositions provided herein include but are not limited to oil, hydrocolloid content, cell mass content (e.g., first and/or second hydrated cell masses) textured protein products with different densities, moisture content, and pH.

As used herein, the term "resilience" refers to a TPA parameter of a cell based meat composition and is calculated by dividing the upstroke energy of the first compression by the downstroke energy of the first compression. Without wishing to be bound by theory, resilience is thought to express how well a cell based meat composition fights to regain its original shape.

As used herein, the term "springiness" refers to a TPA parameter of a cell based meat composition and is calculated as the ratio of the cell based meat composition's height during the second compression and the original compression distance. Without wishing to be bound by theory, springiness is thought to correlate with the ability of a cell based meat composition to spring back after deformation.

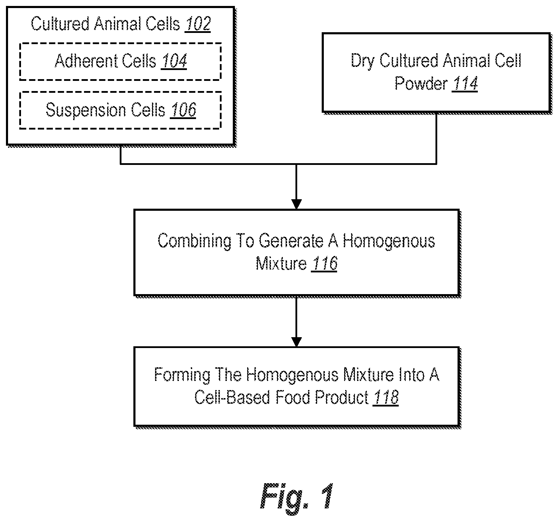

Additional detail will now be provided in relation to illustrative figures portraying example embodiments and implementations of the disclosed methods and apparatuses. For example, FIG. 1 illustrates an overview of a process for generating a homogenous mixture of cultured animal cells and dry cultured animal cell powder to form a comestible cell-based food product. More specifically, as shown in FIG. 1, the comestible cell-based food product can include animal cells 102. To illustrate, in one or more embodiments, the comestible cell-based food products of the disclosure are products produced by culturing of naturally occurring, transgenic, or modified animal cells in culture.

The animal cells can be primary cells and/or cell lines. The methods provided herein are applicable to any metazoan cell in culture. Generally, the animal cells are from any metazoan species whose tissues are suitable for dietary consumption and demonstrate the capacity for skeletal muscle tissue specification. In some embodiments, the animal cells are derived from any non-human animal species intended for human or non-human dietary consumption (e.g., cells of avian, ovine, caprine, porcine, bovine, or piscine origin) (e.g., cells of livestock, poultry, avian, game, or aquatic species).

Additionally, in one or more embodiments, the animal cells are from livestock such as domestic cattle, pigs, sheep, goats, camels, water buffalo, rabbits, and the like. In addition, or in the alternative, in some embodiments, the animal cells are from poultry such as domestic chicken, turkeys, ducks, geese, pigeons, and the like. Further, in one or more embodiments, the animal cells are from game species such as wild deer, gallinaceous fowl, waterfowl, hare, and the like. The animal cells can also be cells from aquatic species or semi-aquatic species harvested commercially from wild fisheries or aquaculture operations, or for sport, including certain fish, crustaceans, mollusks, cephalopods, cetaceans, crocodilians, turtles, frogs, and the like. Additionally, in one or more embodiments, the animal cells are from exotic, conserved or extinct animal species. In some embodiments, the animal cells are from Gallus, Gallus domesticus, Bos taurus, Sous scrofa, Meleagris gallopavo, Anas platyrynchos, Salmo salar, Thunnus thynnus, Ovis aries, Coturnix, Capra aegagrus hircus, or Homarus americanus.

In some embodiments, the animal cells are modifiable by a genetic switch to induce rapid and efficient conversion of the cells to skeletal muscle for cultured production. Additionally, in one or more embodiments, the animal cells are myogenic cells, destined to become muscle, or muscle-like cells. In some embodiments, the myogenic cells are natively myogenic, e.g., myoblasts. Natively myogenic cells include, but are not limited to, myoblasts, myocytes, satellite cells, side population cells, muscle derived stem cells, mesenchymal stem cells, myogenic pericytes, or mesoangioblasts.

Further, in some embodiments, the animal cells are of the skeletal muscle lineage. Cells of the skeletal muscle lineage include myoblasts, myocytes, and skeletal muscle progenitor cells, also called myogenic progenitors that include satellite cells, side population cells, muscle derived stem cells, mesenchymal stem cells, myogenic pericytes, and mesoangioblasts. Additionally, in one or more embodiments, the animal cells are non-myogenic, and such non-myogenic cells can be programmed to be myogenic, for example, the cells may comprise fibroblasts modified to express one or more myogenic transcription factors. In exemplary embodiments, the myogenic transcription factors include MYOD1, MYOG, MYF5, MYF6, PAX3, PAX7, paralogs, orthologs, and genetic variants thereof. In some embodiments, the cells are modified to express one or more myogenic transcription factors as described in a PCT publication, WO/2015/066377,

In some embodiments, the animal cells include a mixture of one or more cell populations described herein. For example, the animal cells can include a mixture of fibrogenic cells and myogenic cells in co-culture. In another example, the animal cells can include a mixture of fibroblasts and myoblasts in co-culture. In some embodiments, the animal cells used for the production of comestible cell-based food products for consumption are a mixture of fibroblasts and myoblasts in a suspension co-culture. In some embodiments the animal cells used for the production of comestible cell-based food products for consumption are a mixture of fibroblasts and myoblasts in an adherent co-culture. In some embodiments, the co-culture can further comprise adipocytes.

In some embodiments, the animal cells are genetically modified to inhibit a pathway, e.g., the HIPPO signaling pathway. Exemplary methods to inhibit the HIPPO signaling pathway as described in a PCT Application No. PCT/US2018/031276, Further, in one or more embodiments, the cells are modified to express telomerase reverse transcriptase (TERT) and/or inhibit cyclin-dependent kinase inhibitors (CKI). Additionally, in some embodiments, the cells are modified to express TERT and/or inhibit cyclin-dependent kinase inhibitors as described in a PCT publication, WO 2017/124100,

Additionally, in one or more embodiments, the animal cells are modified to express glutamine synthetase (GS), insulin-like growth factor (IGF), and/or albumin. Exemplary methods of modifying cells to express GS, IGF, and/or albumin are described in a PCT Application No. PCT/US2018/042187

Additionally, it will be appreciated that the animal cells can comprise any combination of the modifications described herein. Similarly, in one or more embodiments, the animal cells can include a combination of the various cell types described herein.

As shown in FIG. 1, the cultured animal cells 102 can optionally include adherent cells 104 and/or suspension cells 106. In one or more embodiments, the comestible cell-based food product includes adherent cells in combination with dry cultured animal cell powder, suspension cells in combination with dry cultured animal cell powder, or both adherent cells and suspension cells in combination with dry cultured animal cell powder. Additionally, the adherent cells 104 and/or the suspension cells 106 can include any of the variety of cell types and/or modifications described above with regard to the animal cells.

In some embodiments, the cultured animal cells 102 include the adherent cells 104 and the suspension cells 106, and comprise a mixture of fibroblasts and myoblasts, wherein the ratio of the fibroblasts to myoblasts (designated as F and M) ranges from about 5F:95M to about 95F:5M. In exemplary embodiments, the ratio of the fibroblasts to myoblasts is about 5F:95M, 10F:90M, 15F:85M, 20F:80M, 25F:75M, 30F:70M, 35F:65M, 40F:60M, 45F:55M, 50F:50M, 55F:45M, 60F:40M, 65F:35M, 70F:30M, 75F:25M, 80F:20M, 85F:15M, 90F:10M, or even about 95F:5M.

One or more embodiments involve culturing the adherent cells 104 and/or the suspension cells 106 in a cultivation infrastructure. As referred to herein, a cultivation infrastructure refers to the environment in which the cells are cultured or cultured to provide a two-dimensional or three-dimensional product for consumption. A cultivation infrastructure may be a roller bottle, a tube, a cylinder, a flask, a petri-dish, a multi-well plate, a dish, a vat, an incubator, a bioreactor, an industrial fermenter, etc.

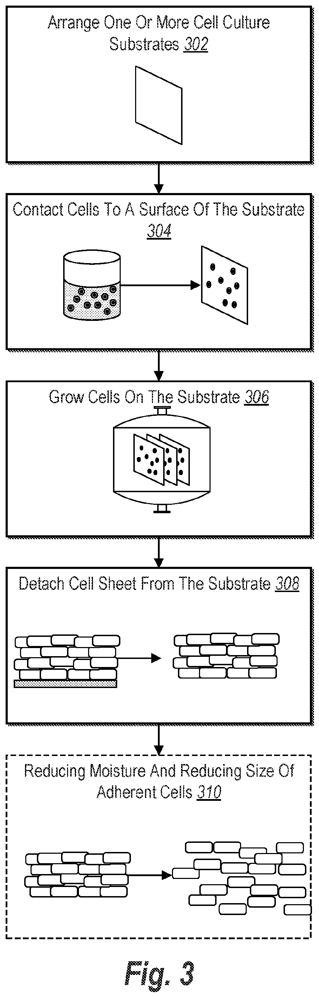

As mentioned above, the animal cells 102 can include adherent cells 104. One or more embodiments involve preparing the adherent cells 104 by seeding cells to a surface of a substrate and growing the cells on the substrate to form cell sheets. The methods further involve detaching a resulting cell sheet from the substrate. More detail regarding cultivating adherent cells is provided in greater detail in relation to FIG. 3.

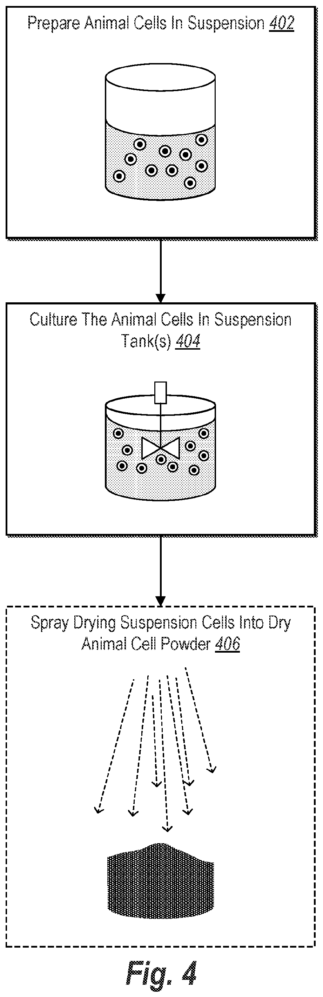

Additionally, in some embodiments, animal cells 102 include the suspension cells 106 cultured in an at least partially liquid growth medium in which cells grow, multiply, and/or maintain nourishment. In some embodiments, involve growing suspension cells 106 in a suspension culture. For example, one or more embodiments involve growing suspension cells 106 in a shake flask. To illustrate, in one or more embodiments, the product of the culture is centrifuged, yielding a cell pellet. Some embodiments involve growing suspension cells 106 while free-floating in the culture media. More detail regarding cultivating suspension cells is provided in greater detail in relation to FIG. 4.

As shown in FIG. 1, one or more embodiments involve optional pre-processing 108 of the cultured animal cells 102. In one or more embodiments, the pre-processing 108 optionally includes moisture adjustment 110 and/or size reduction 112. In one or more embodiments, moisture adjustment 110 is performed by vacuum drying the cultured animal cells 102. In addition, in some embodiments, size reduction 112 comprises reducing a size of the animal cells 102 via chopping or other technique.

For example, act 108 can include cutting cultured cell tissue into fine pieces. In particular, part of act 108 involves finely chopping cultured cell tissue into fine pieces. In some embodiments, act 108 includes size reducing cultured cell tissue by chopping, dicing, cutting, or pulverizing the grown cells. As a result of performing the act 108, at least a portion of long fibers are broken down into shorter and more compact fibers. In some embodiments, the act 108 further includes mixing the fine pieces with each other (e.g., using tines or an extruder) while aligning meat fibers. In some embodiments, the act 108 is performed by coarsely chopping or cutting the cultured cell tissue into squares or rectangles having a threshold length. In one or more embodiments, the small segments are rectangular cuboids having an approximate length, width, and height. For example, in at least one embodiment, the length of the small segments equals 9 cm, the width equals 3 cm, and the height equals ½ cm. Alternatively, reducing the size of the cultured cell tissue involves forming irregular shaped and sized segments or agglomerations of cultured cell tissue.

Additionally, act 108 can involve reducing moisture content of (e.g., partially drying) a comestible cell-based meat product after being harvested from a bioreactor or suspension tank. For example, in one or more embodiments, act 108 involves vacuum drying that uses a combined pressure-temperature manipulation to evaporate a portion of the moisture content from a comestible cell-based meat product. In doing so, the vacuum drying method can concentrate protein in the comestible cell-based meat product, as well as minerals and other non-liquid components. In one or more embodiments, vacuuming drying is done at refrigeration temperatures to preserve cellular structure of the comestible cell-based meat product and facilitate safe processing according to various rules and regulations for raw meat sale, distribution, and consumer consumption. In addition, lowered environmental pressure provides reduced drying times to achieve a particular moisture content criteria or threshold mass of the comestible cell-based meat product.

As also shown in FIG. 1, in one or more embodiments, the comestible cell-based food product includes dry cultured animal cell powder 114. In one or more embodiments, the dry cultured animal cell powder is prepared via various methods for drying and powdering cultured animal cells (e.g., adherent cells and/or suspension cells). To illustrate, in one or more embodiments, the dry cultured animal cell powder is prepared by spray drying suspension cells or by drying and powdering adherent cells.

Further, as shown in FIG. 1, the comestible cell-based food product is prepared by performing an act 116 of combining the cultured animal cells 102 and the dry cultured animal cell powder 114 to generate a homogenous or nearly homogenous mixture. More specifically, one or more embodiments include combining the animal cells 102 and the dry cultured animal cell powder 114 to generate a homogenous mixture. As discussed above, the homogenous mixture can include adherent cells 104 and dry cultured animal cell powder 114, suspension cells 106 and dry cultured animal cell powder 114, or all three of adherent cells 104, suspension cells 106, and dry cultured animal cell powder 114. To illustrate, in one or more embodiments, the animal cells 102 and the dry cultured animal cell powder 114 are mixed under a vacuum to create a homogenous mixture for further processing.

Optionally, as part of act 116, the method can include adding other functional ingredients/additives to the homogenous mixture. For example, act 116 can involve adding other nutrients, such as vitamins, to increase the nutritional value of the cell-based product. For example, this may be achieved through the exogenous addition of the nutrients to the growth medium or through genetic engineering techniques. Additionally, phosphates may be added to help extract the protein from the cell mixture and to increase the moisture retention in the final product, which helps emulate the texture of conventional meat. Stabilizing agents, such as starches or gums, may be added to help bind water to the composition and to improve the overall texture and water content of the composition. Spices and flavoring agents may be added to the final mixture to help build base flavor profiles similar to conventional meat. Nutrients, such as common vitamins and minerals, may be added to the composition in order to better enhance the nutritional value of the final product.

In some embodiments, act 116 also involves adding a stabilizing agent to the homogenous mixture that helps the composition maintain its shape during the forming and cooking of the composition. Exemplary stabilizers include, but are not limited to starches, gums, polysaccharides, protein concentrates, protein isolates, and other commonly used gelling agents and thickeners. In some embodiments, the composition may also contain one or more components that help enhance flavor, nutritional value, and/or texture of the final product, such as phosphates, spices, preservatives, artificial flavoring agents, vitamins, or minerals.

Additionally, as shown in FIG. 1, the comestible cell-based food product is prepared by performing an act 118 of forming the homogenous mixture into a cell-based food product. To illustrate, in one or more embodiments, the raw mixture is formed into desired shapes or formats with the assistance of stuffers, formers, molds, or other process-appropriate equipment. In some embodiments, a comestible cell-based food product is ready for immediate packaging use following formation. In addition, or in the alternative, the comestible cell-based food product is cooked before packaging or storage.

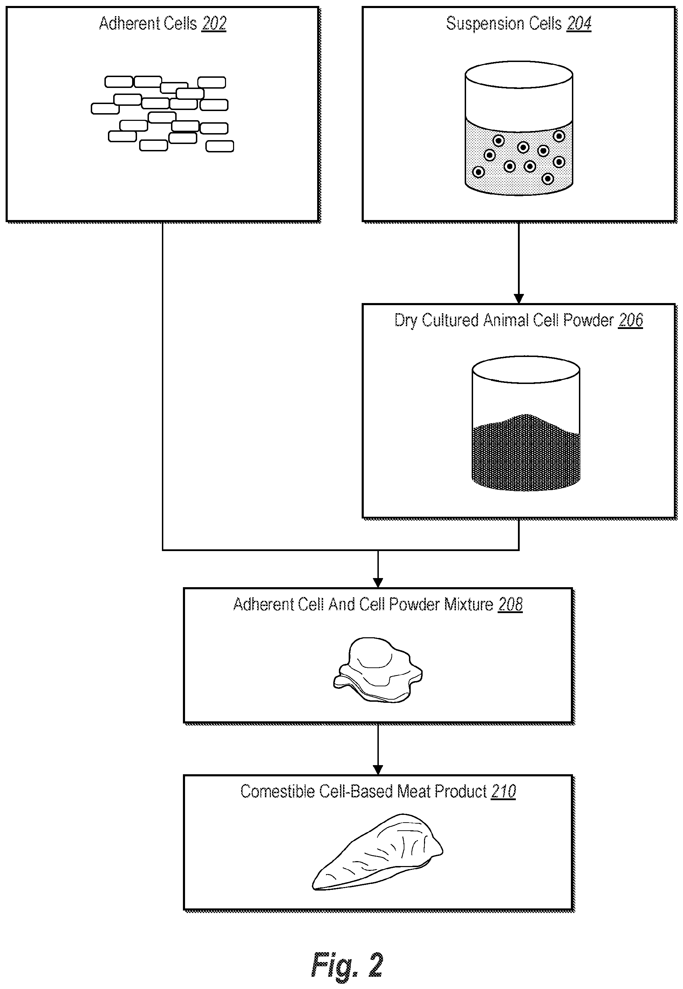

As mentioned above, one or more embodiments include cultured adherent cells and/or cultured suspension cells mixed with dry cultured animal cell powder. FIG. 2 illustrates a process for combining adherent cells 202 and dry cultured animal cell powder 206 to form an adherent cell and tissue cell powder mixture 208. As will be discussed in greater detail below with regard to FIG. 3, in one or more embodiments involve preparing adherent cells 202 by contacting cells to a surface of a substrate, growing the cells on the substrate, detaching a resulting cell sheet from the substrate, and optionally reducing the moisture content and/or size of the adherent cells.

Further, as shown in FIG. 2, one or more embodiments involve preparing the dry cultured animal cell powder from suspension cells 204. To illustrate, one or more embodiments involve dehydrating animal cells via forced air drying, spray drying, and/or vacuum drying to form the dry cultured animal cell powder 206. In some embodiments, the dry cultured animal cell powder 206 is prepared by spray drying the suspension cells 204. Further, one or more alternative embodiments involve forming the dry cultured animal cell powder 206 by drying and powdering adherent cells. However, it will be appreciated that the dry cultured animal cell powder can also be prepared in a variety of drying and powdering processes.

As shown in FIG. 2, the comestible cell-based food product can include the adherent cell and tissue cell powder mixture 208. To illustrate, in one or more embodiments, the comestible cell-based food product is prepared by mixing suspension cells and/or adherent cells with dry cultured animal cell powder to form a homogenous mixture. In some embodiments, the homogenous mixture is prepared under a vacuum.

In some embodiments, the comestible cell-based food product generally includes about 25 g to about 95 g by weight of amino acids per 100 g dry mass. Additionally, the comestible cell-based food product generally has a moisture content of 60% to 95%. Differing amounts of adherent and suspension cells can then be mixed until a desired ratio of the animal cells 102 (e.g., the adherent cells 104 and/or the suspension cells 106) and the dry cultured animal cell powder 114 is formed. Generally, higher proportions of suspension cells in comparison to adherent cells may be used to create a softer, fattier product while higher proportions of adherent cells in comparison to suspension cells may be used to create a leaner product.

Similarly, higher proportions of dry cultured animal cell powder 114 can be used to create a leaner, harder, and less adhesive product. The dry cultured animal cell powder contributes to the final product by allowing for the cells in the cell mixture to better adhere to each other in order to form and retain a desired shape.

In one embodiment, the comestible cell-based meat product 210 may be prepared from a mixture of adherent tissues and suspension cells in order to better emulate the conventional taste, texture, and flavor of natural meat. To illustrate, in one or more embodiments, the homogenous mixture is composed of 0.1%-30% of dry cultured animal cell powder and 70%-99.9% of adherent cells. Additionally, in some embodiments, the homogenous mixture is composed of 0.1%-40% of dry cultured animal cell powder and 60%-99.9% of suspension cells. Additionally, in some embodiments, the homogenous mixture is composed of 0.1%-40% of dry cultured animal cell powder, .1%-99.8% of suspension cells, and 0.1%-99.8% of adherent cells.

In one embodiment, the comestible cell-based meat product 210 is composed of 80% adherent cells with 20% dry cultured animal cell powder. In another embodiment, the comestible cell-based meat product 210 is composed of 95% adherent cells with 5% dry cultured animal cell powder. In yet another embodiment, the comestible cell-based meat product 210 is composed of 99% adherent cells with 1% dry cultured animal cell powder. In another embodiment the comestible cell-based meat product 210 may be composed of 97% adherent cells with 3% dry cultured animal cell powder. In another embodiment the comestible cell-based meat product 210 is composed of 95% adherent cells with 5% dry cultured animal cell powder. In another embodiment the comestible cell-based meat product 210 is composed of 90% adherent cells with 10% dry cultured animal cell powder. In another embodiment, the comestible cell-based meat product 210 is composed of 80% adherent cells with 20% dry cultured animal cell powder. In another embodiment the comestible cell-based meat product 210 is composed of 70% adherent cells with 30% dry cultured animal cell powder.

In another embodiment, the comestible cell-based meat product 210 is composed of 60% suspension cells with 40% dry cultured animal cell powder. In another embodiment, the comestible cell-based meat product 210 is composed of 65% suspension cells with 35% dry cultured animal cell powder. In yet another embodiment, the comestible cell-based meat product 210 is composed of 70% suspension cells with 30% dry cultured animal cell powder. In another embodiment the comestible cell-based meat product 210 may be composed of 75% suspension cells with 25% dry cultured animal cell powder. In another embodiment the comestible cell-based meat product 210 is composed of 80% suspension cells with 20% dry cultured animal cell powder. In another embodiment the comestible cell-based meat product 210 is composed of 85% suspension cells with 15% dry cultured animal cell powder. In another embodiment the comestible cell-based meat product 210 is composed of 90% suspension cells with 10% dry cultured animal cell powder.

As discussed above, the comestible cell-based food product can include adherent cells. FIG. 3 illustrates a process for preparing adherent cells. More specifically, in one or more embodiments, the adherent cells are prepared by performing an act 302 of arranging one or more cell culture substrates. In some embodiments, the cultivation infrastructure for the adherent cells 104 includes a substrate. More specifically, in one or more embodiments, the cultivation infrastructure for the adherent cells 104 may comprise a permeable substrate (e.g., permeable to physiological solutions) or an impermeable substrate (e.g., impermeable to physiological solutions). The substrate for the adherent cells 104 can be flat, concave, or convex. Additionally, the substrate for the adherent cells 104 can be textured so as to promote cell growth.

Further, as shown in FIG. 3, the adherent cells can be prepared by performing an act 304 of contacting cells to a surface of the substrate. To illustrate, in one or more embodiments, the adherent cells are prepared by contacting cell culture substrates to a cultivation infrastructure, including within a bioreactor.

In some embodiments, the culturing of adherent cells 104 in the cultivation infrastructure can induce the production of extracellular matrix (ECM). Indeed, in one or more embodiments, the ECM can act as an autologous scaffold to direct three-dimensional cellular growth. For example, in some embodiments, the ECM can direct attachment, proliferation, and hypertrophy of cells on a plane perpendicular to the substrate. In addition, or in the alternative, in some embodiments, the cultivation infrastructure may not comprise an exogenously added scaffold to promote self-assembly of a three-dimensional cellular biomass. In some embodiments, the cultivation infrastructure may not comprise exogenous scaffolds such as a hydrogel or soft agar.

As mentioned above, adherent cells can be grown to form a cell sheet. Table 1 provides exemplary culture methods for the various cell-based food products.

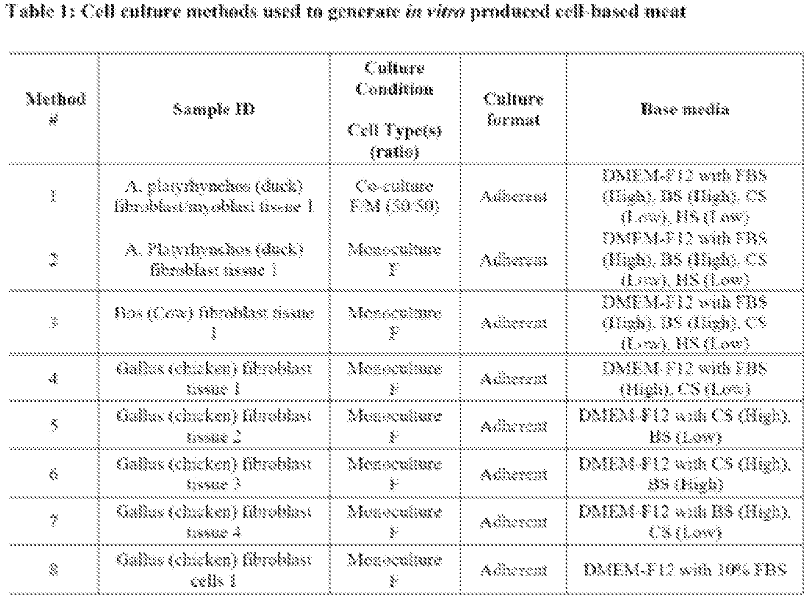

In one or more embodiments, for the cell culture methods of table 1, the media is substantially free of serum or other components derived from an animal. Accordingly, an exemplary method of producing cell-based meat comprises: (a) providing fibroblasts and/or myoblasts from a non-human organism; (b) culturing the fibroblasts and/or myoblasts in media under conditions under which the fibroblasts and/or myoblasts grow in either suspension culture or adherent culture, wherein the media is substantially free of serum and other components derived from an animal.

Additionally, as shown in FIG, 3, in one or more embodiments, the adherent cells are prepared by performing an act 306 of growing cells on the substrate. In one or more embodiments, the adherent cells 104 are grown on a suitable substrate that is specifically treated to allow cell adhesion and spreading, such as a surface located within a sterile bioreactor. More specifically, the culturing conditions for the generation of the animal cells 102 for a comestible cell-based food product are generally aseptic and sterile. In some cases, cells are injected into a cultivation tank, such as an adherent bioreactor. The cultivation tank contains the substrate (e.g., metal planks or sheets) to which cells can adhere. The cells are flowed through the cultivation tank to allow cells to adhere to the substrate over time.

Before seeding the cells onto the substrate, in some embodiments, the disclosed methods include preparing the substrate, such as by adding or flowing over adherent media to increase cell adherence to the substrate. As suggested above, in some implementations, the substrate is located within a cultivation tank that is a sterile environment. To prepare the substrate in a cultivation tank, the disclosed methods can include adding adherent media. The adherent media can be low in calcium to limit cellular clumping so the cells spread out evenly across the substrate. The adherent media further facilitates attachment by the cells to the substrate. In some implementations, preparing the substrate further includes adding conditioning media and bringing the conditioning media up to temperature. The conditioning media further prepares the substrate by controlling pH, carbon dioxide, and oxygen levels within the cultivation tank.

The disclosed methods include growing the cells into a cellular tissue. Generally, the seeded cells (including the seeded initial cells and the previously unlanded cells) are grown in conditions that allow the formation of cellular tissue for a formation period. In some cases, the formation period can equal 4-14 days. During the formation period, cells may be provided with additional nutrients, media, growth factors, and other supplements to promote cellular growth. For example, the disclosed methods can include providing growth media on day 1. The growth media can include growth factors and beneficial proteins. At nutrient intervals (e.g., every three days) during the formation period, additional feeds, amino acids, proteins, vitamins, minerals, and growth factors may be added to the cultivation tank to support growth in the seeded cells. Additionally, or alternatively, the disclosed methods include adding pre-harvest media before harvest. For instance, three days before harvest, a pre-harvest media including yeast concentrate may be added to the cultivation tank.

In one or more embodiments, the adherent cells include cellular tissue of cultured meat gathered into a collective or agglomerated mass, including via growth on a substrate. In one or more embodiments, the cultivation infrastructure for cultivating the adherent cells has a three-dimensional structure or shape for cultivating a monolayer of adherent cells. Additionally, in some embodiments, the cultivation infrastructure can promote the adherent cells to form a three-dimensional growth of metazoan cells in the cultivation infrastructure to provide a scaffold-less self-assembly of a three-dimensional cellular biomass.

In some embodiments, the adherent cells are grown on a three-dimensional cultivation infrastructure. The three-dimensional cultivation infrastructure may be sculpted into different sizes, shapes, and forms, as desired, to provide the shape and form for the adherent cells 104 to grow and resemble different types of muscle tissues such as steak, tenderloin, shank, chicken breast, drumstick, lamb chops, fish fillet, lobster tail, etc. The three-dimensional cultivation infrastructure may be made from natural or synthetic biomaterials that are non-toxic so that they may not be harmful if ingested. Natural biomaterials may include, for example, collagen, fibronectin, laminin, or other extracellular matrices. Synthetic biomaterials may include, for example, hydroxyapatite, alginate, polyglycolic acid, polylactic acid, or their copolymers. The three-dimensional cultivation infrastructure may be formed as a solid or semisolid support.

A cultivation infrastructure can be of any scale, and support any volume of cellular biomass and culturing reagents. In some embodiments, the cultivation infrastructure ranges from about 10 µL to about 100,000 L. In exemplary embodiments, the cultivation infrastructure is about 10 µL, about 100 µL, about 1 mL, about 10 mL, about 100 mL, about 1 L, about 10 L, about 100 L, about 1000 L, about 10,000 L, or even about 100,000L.

In one or more embodiments, the comestible cell-based food product, unless otherwise manipulated to include, does not include vascular tissues, such as veins and arteries, whereas conventional meat does contain such vasculature, and contains the blood found in the vasculature. Accordingly, in some embodiments, the comestible cell-based food product does not comprise any vasculature.

Likewise, comestible cell-based food product, although composed of muscle or muscle-like tissues, unless otherwise manipulated to include, does not comprise functioning muscle tissue. Accordingly, in some embodiments, the cell-based meat does not comprise functioning muscle tissue. It is noted that features such as vasculature and functional muscle tissue can be further engineered into the cell-based meat, should there be a desire to do so.

Also, as shown in FIG, 3, the adherent cells can be prepared by performing an act 308 of detaching a cell sheet from the substrate. In one or more embodiments, the adherent cells are harvested by detaching a cell sheet from the substrate. In particular, the adherent cells are harvested based on various factors. The adherent tissue may be harvested after a proliferation time period. For example, the adherent cells are harvested after the cells have been growing for anywhere between 4 and 14 days. In another example, the adherent cells are harvested based on completing a proliferation phase. More specifically, the adherent cells may be harvested when the cell sheet starts contracting and stops growing. For instance, the cell sheet may begin to detach from the substrate. In one or more embodiments, the cell sheet is detached from the substrate after adding pre-harvest media before harvest.

Additionally, the dry cultured animal cell powder can be prepared from adherent cells by drying an adherent cell sheet and powdering to form a dry cultured animal cell powder. More specifically, the adherent cells can be dried to a threshold moisture percentage. Additionally, in one or more embodiments, the dry cultured animal cell powder is prepared by powdering the dried adherent cells. To illustrate, the dried adherent cells can be physically powdered or pulverized by a variety of crushing or grinding methods.

As shown by FIG. 3, the method can optionally involve reducing the moisture and/or size of an agglomeration (e.g., the cell sheet) of adherent cells 310. As discussed above, act 310 can involve reducing a moisture content in the adherent cells by vacuum drying the adherent cells. In addition, in some embodiments, act 310 involves reducing a size of the animal cells 102 via chopping or other technique.

As also mentioned above, in one or more embodiments, the comestible cell-based food product includes suspension cells. FIG. 4 illustrates a process for generating suspension cells. More specifically, as shown in FIG. 4, the suspension cells can be prepared by performing an act 402 of preparing animal cells in suspension. To illustrate, animal cells are suspended in cell media. In one implementation, cells are thawed and grown in smaller flasks before transfer to the suspension tanks. The cells are injected into the suspension tanks with cell culture media to proliferate.

Additionally, in some embodiments, the suspension cells are prepared by performing an act 404 of culturing the animal cells in suspension tank(s). More specifically, in one or more embodiments, suspension tanks hold suspension cultures and provide a vessel in which cells may be passaged and grown. To illustrate, a suspension tank can hold cells and cell media to help cells grow. In some embodiments, a suspension tank comprises a plurality of vessels for scaling up and passaging of cells to stimulate growth.

In one or more embodiments, the suspension cells can be grown in various suspension tanks, and each suspension tank of the suspension tanks may hold different types of cells. To illustrate, the suspension tanks may hold fibroblasts, myocytes, and adipocytes. However, the suspension cells may also be grown in fewer or more suspension tanks. Furthermore, the suspension tanks may or may not be connected to each other.

In one or more embodiments, suspension cells are injected from a suspension tank into a media reservoir. The media reservoir includes an agitator for keeping cells solubilized in cell media. To illustrate, the agitator agitates the cell and cell media mixture to keep the cells uniform through the cell media. By maintaining uniformity within the cell mixture, the apparatus improves the evenness of distribution of cells across substrates within the cultivation tank.

As mentioned, the disclosed methods comprise growing cells in suspension. Generally, growing cells in suspension is faster and more economical relative to adherent tissue formation. Thus, in some implementations, the disclosed methods comprise scaling up the growth of cells in suspension rather than using an adherent culture for initially growing cells before seeding.

Generally, in one or more embodiments, cells are grown in a suspension culture to a threshold cell density. In one example, cells are grown in a suspension 212 using a 2-4-day passage cadence. Generally, passaging cells comprises transferring cells from old cell culture media to fresh cell culture media. Passaging the cells may comprise moving the cells into larger containers to provide additional room for cellular growth. In one or more implementations, the cells are passaged at a cadence of 2-4 days with an approximately 20-hour (20.202 hours +/- 1.97 hours, 16.9-25.6-hour range) population doubling time. In some embodiments, the cells are duplicated until they reach a threshold cell suspension density. In one example, the threshold cell suspension density equals a viable cell density of approximately 3-5 million cells/mL in suspension. But the threshold cell suspension density may equal more than 5 million cells/mL in suspension, including a threshold approximately 20 million cells/mL in suspension. In some embodiments, the disclosed methods comprise duplicating the cells until they have completed a proliferation stage. For example, the disclosed methods may include measuring ammonia, oxygen consumption, pH, and lactate to determine whether cells are actively proliferating.

Further, in one or more embodiments, the dry cultured animal cell powder is prepared by performing an act 406 of spray drying suspension cells into dry cultured animal cell powder. To illustrate, in one or more embodiments, suspension cells are sprayed and allowed to dry into a powder. In some embodiments, the suspension cells are sprayed in a drying chamber with heated air running through. Further, in one or more embodiments, the dried cells are collected via an attached powder collector.

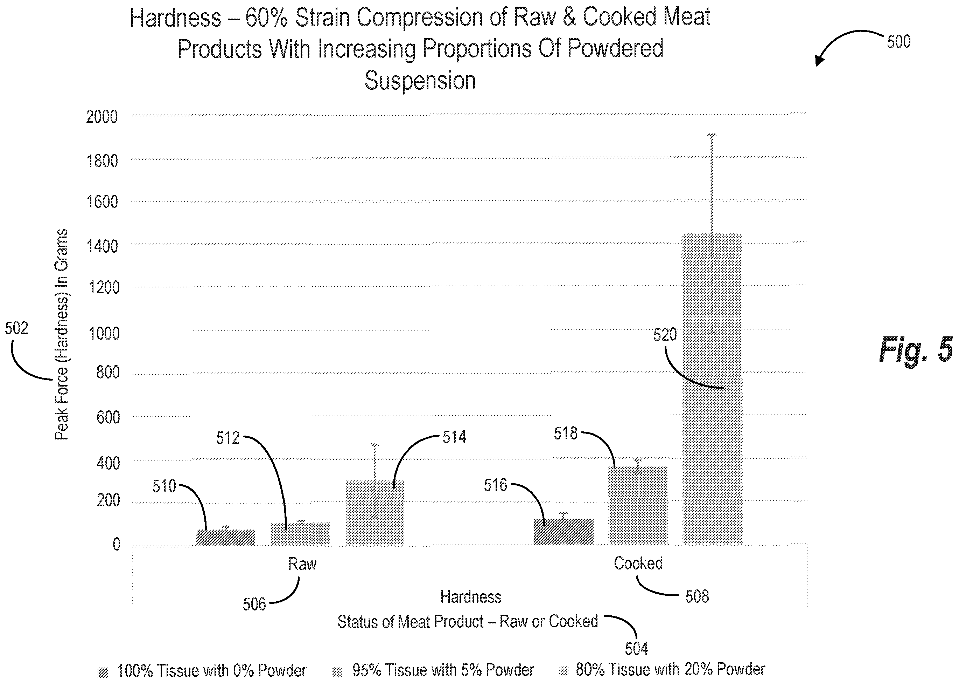

As mentioned above, generating the comestible cell-based food product including dry cultured animal cell powder can improve the texture of the comestible cell-based food product by causing the hardness and adhesiveness of the comestible cell-based food product to match more closely that of conventional meat products. FIGS. 5-8 illustrate charts of the hardness or adhesiveness of various comestible cell-based food products prepared from adherent cells and dry cultured animal cell powder. In particular, the comestible cell-based food product tested in connection with FIGS. 5-8 comprised cultured adherent chicken cells with dehydrated cultured suspension chicken cell powder.

FIG. 5 illustrates a graph of hardness at 60% strain compression of raw and cooked comestible cell-based food products with various proportions of dry cultured animal cell powder in accordance with one or more embodiments. More specifically, the graph 500 includes a y-axis 502 showing peak force (hardness) in grams and an x-axis 504 showing the observed values of the meat product, raw or cooked. More specifically the graph 500 includes the raw samples 506 and the cooked samples 508. The raw and cooked comestible cell-based food products are compared with conventional slaughtered chicken both raw and cooked. For the testing of the conventional slaughtered chicken, two types of test samples were used, each in replicates of 5. The first type of test sample comprised slaughtered ground chicken of mixed origin (i.e., dark, white, and fat). The second type of test sample comprised slaughtered pure breast ground chicken (i.e., no fat or dark meat). Both types of test samples were ground and formed for use in the experiment without the addition of any other ingredients. For the data we collected on conventional slaughtered chicken, the conventional slaughtered chicken was ground, formed, and cooked to 165 degrees Fahrenheit with no other ingredients added. One will appreciate, however, that conventional slaughtered chicken hardness and adhesiveness can be manipulated by, for example: by cooking temp (e.g., slowing cook produces a cooked product that is less hard), salt (generally more salt results in reduced hardness), amount of mixing (more mixing results in reduced hardness), fat content (generally higher fat results in a reduced hardness), moisture content (e.g., brining results in reduced hardness due to more moisture and salt entering into the product), mechanical tenderizing (e.g., stabbing with needles or hitting with hammers to reduce hardness), pH adjustment (e.g., buttermilk brine - acid can help reduce hardness), or acid induced denaturation (ceviche "cooked" w/ pH adjustment to be less hard).

As shown in FIG. 5, bar 510 corresponds to a raw comestible cell-based food product with 100% adherent cells and shows a hardness of approximately 72.9 grams of peak force. By comparison conventional slaughtered raw chicken comprises approximately 82.5 grams of peak force. Thus, a raw comestible cell-based food product with 100% adherent cells can have a texture that is noticeably different than conventional slaughtered raw chicken. This difference in texture can be due at least in part to a difference in hardness. FIG. 5 furthermore shows bar 512 corresponding to a raw comestible cell-based food product with 95% adherent cells and 5% dry cultured animal cell powder shows a hardness of approximately 105.8 grams of peak force. Further, bar 514 corresponding to a raw comestible cell-based food product with 80% adherent cells and 20% dry cultured animal cell powder shows a hardness of approximately 298.2 grams of peak force. FIG. 5 illustrates how adding dry cultured animal cell powder can modify or tailor the hardness of a raw comestible cell-based food product. In particular, FIG. 5 illustrates how, in one or more embodiments, adding dry cultured animal cell powder to a comestible cell-based food product can tailor the hardness to be more consistent with conventional slaughtered raw meat. In particular, one or more embodiments involves adding an amount of dry cultured animal cell powder to a comestible cell-based food product to tailor the hardness to be consistent (i.e., within acceptable tolerance) with that of conventional slaughtered raw meat. One will appreciate in light of the disclosure herein that having a hardness closer to that of conventional slaughtered meat provides consumers with an eating experience that they expect. Too large of a deviation of hardness from conventional slaughtered meat may lead to perceptions of a comestible cell-based food product being too soft or too hard. While other properties effect texture in addition to hardness, having deviations larger than an acceptable tolerance can lead to unmet culinary expectations. On the other hand, the closer the hardness of a comestible cell-based food product to conventional slaughtered meat, the more likely the consumer will have a positive eating experience.

Additionally, as shown in FIG. 5, bar 516 corresponding to a cooked comestible cell-based food product with 100% adherent cells shows a hardness of approximately 121.1 grams of peak force. By comparison conventional slaughtered cooked chicken comprises approximately 893.9 grams of peak force. In other words, the cooked comestible cell-based food product with 100% adherent cells has a hardness that is approximately 13.55 percent of corresponding conventional slaughtered cooked meat. Thus, a cooked comestible cell-based food product with 100% adherent cells can have a texture that is noticeably different than conventional slaughtered cooked chicken. This difference in texture can be due at least in part to a difference in hardness and can result in a noticeable difference in eating experience. FIG. 5 includes bar 518 corresponding to a cooked comestible cell-based food product with 95% adherent cells and 5% dry cultured animal cell powder and shows a hardness of approximately 362.1 grams of peak force. Further, bar 520 corresponding to a cooked comestible cell-based food product with 80% adherent cells and 20% dry cultured animal cell powder and shows a hardness of approximately 1441.0 grams of peak force. Thus, FIG. 5 illustrates how adding dry cultured animal cell powder can modify or tailor the hardness of a cooked comestible cell-based food product. In particular, FIG. 5 illustrates how, in one or more embodiments, adding dry cultured animal cell powder to a comestible cell-based food product can tailor the hardness to be more consistent with conventional slaughtered cooked meat. In particular, one or more embodiments involves adding an amount of dry cultured animal cell powder to a comestible cell-based food product to tailor the hardness to be consistent (i.e., within acceptable tolerance) with that of conventional slaughtered cooked meat. For example, in one or more embodiments, adding dry cultured animal cell powder can tailor the hardness of a cooked comestible cell-based meat product to have a percent difference of less than 30 percent, 20 percent, 10 percent, or 5 percent compared to the hardness of a corresponding conventional slaughtered cooked meat.

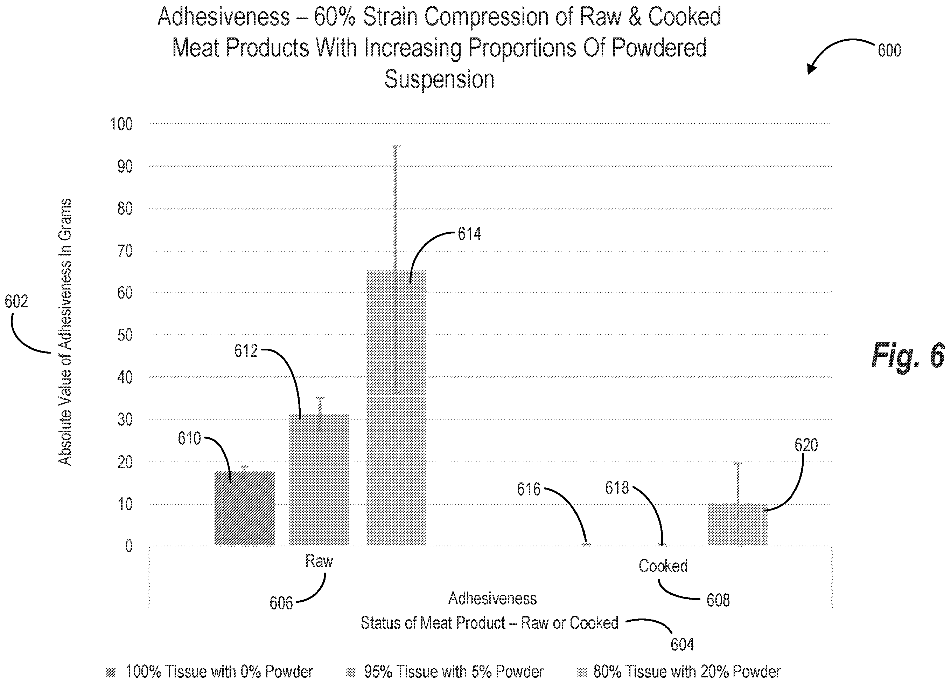

FIG. 6 illustrates adhesiveness at 60% strain compression of raw and cooked comestible cell-based food products with various proportions of dry cultured animal cell powder in accordance with one or more embodiments. More specifically, the graph 600 includes a y-axis 602 showing the absolute value of adhesiveness in grams and an x-axis 604 showing the status of the meat product, raw or cooked. More specifically the graph 600 includes the raw samples 606 and the cooked samples 608.

As shown in FIG. 6, a bar 610 corresponding to a raw comestible cell-based food product with 100% adherent cells shows an absolute value of adhesiveness of approximately 17.8 grams. By comparison conventional slaughtered raw chicken was observed to have an absolute value of adhesiveness of approximately 16.6 grams. Additionally, bar 612 corresponding to a raw comestible cell-based food product with 95% adherent cells and 5% dry cultured animal cell powder shows an absolute value of adhesiveness of approximately 31.4 grams. Further, bar 614 corresponding to a raw comestible cell-based food product with 80% adherent cells and 20% dry cultured animal cell powder shows an absolute value of adhesiveness of approximately 65.4 grams.

As also shown in FIG. 6, a bar 616 corresponding to a cooked comestible cell-based food product with 100% adherent cells shows an absolute value of adhesiveness of approximately 0.3 grams. By comparison conventional slaughtered cooked chicken comprises an absolute value of adhesiveness of approximately 12.5 grams of peak force. Thus, a cooked comestible cell-based food product with 100% adherent cells can have a texture that is noticeably different than conventional slaughtered cooked chicken. This difference in texture can be due at least in part to a difference in adhesiveness and can result in a noticeable difference in eating experience. Additionally, a bar 618 corresponding to a cooked comestible cell-based food product with 95% adherent cells and 5% dry cultured animal cell powder shows an absolute value of adhesiveness of approximately 0.4 grams. A bar 620 corresponding to a cooked comestible cell-based food product with 80% adherent cells and 20% dry cultured animal cell powder shows an absolute value of adhesiveness of approximately 10.1 grams.

FIG. 6 illustrates how adding dry cultured animal cell powder can modify or tailor the adhesiveness of a cooked comestible cell-based food product. In particular, FIG. 6 illustrates how, in one or more embodiments, adding dry cultured animal cell powder to a comestible cell-based food product can tailor the adhesiveness to be more consistent with conventional slaughtered cooked meat. In particular, one or more embodiments involves adding an amount of dry cultured animal cell powder to a comestible cell-based food product to tailor the average adhesiveness to be consistent (i.e., within acceptable tolerance) with that of conventional slaughtered cooked meat. One will appreciate in light of the disclosure herein that having an adhesiveness closer to that of conventional slaughtered meat provides consumers with an eating experience that they expect. Too large of a deviation of adhesiveness from conventional slaughtered meat may lead to perceptions of a comestible cell-based food product being too sticky or too non-adhesive. While other properties effect texture in addition to adhesiveness, having deviations larger than an acceptable tolerance can lead to unmet culinary expectations. On the other hand, the closer the adhesiveness of a comestible cell-based food product to conventional slaughtered meat, the more likely the consumer will have a positive eating experience.

As illustrated by FIG. 6, the percent composition of spray dried cells correlates with increased average adhesiveness, however a high percent spray dried cell replicates captured here showed a large standard deviation that overlaps with low percent spray dried cells embodiments, which indicates the potential need for further refinement. Nevertheless, the replicates across all percent composition spray dried cells illustrate that higher adhesiveness scores are only enabled at higher percent composition spray dried cells. For example, in one or more embodiments, adding dry cultured animal cell powder can tailor the adhesiveness of a cooked comestible cell-based meat product to have a percent difference of less than 30 percent, 20 percent, 10 percent, or 5 percent compared to the adhesiveness of a corresponding conventional slaughtered cooked meat.

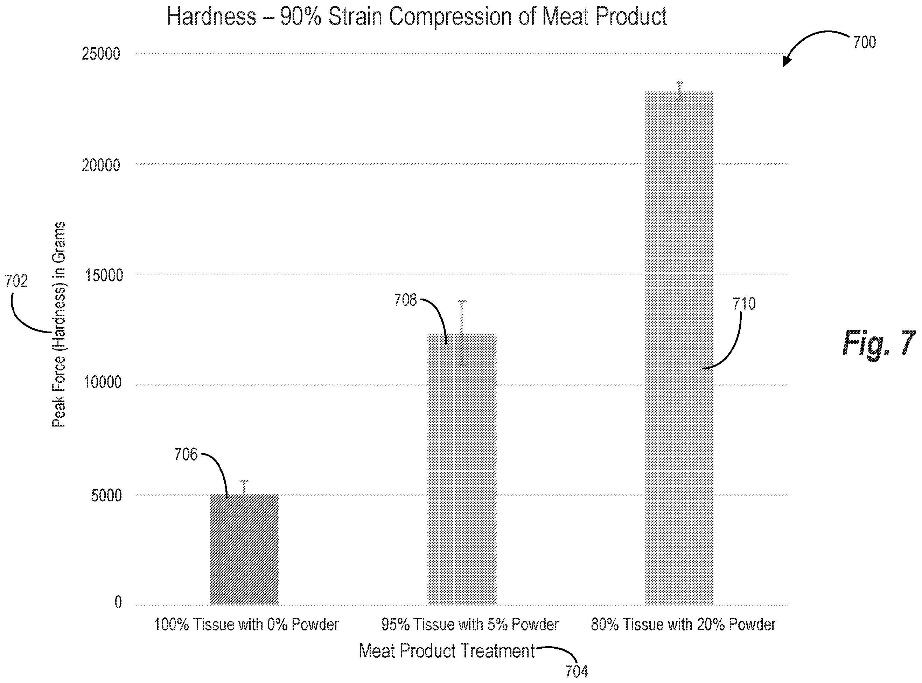

FIG. 7 illustrates hardness at 90% strain compression of raw comestible cell-based food products with various proportions of dry cultured animal cell powder in accordance with one or more embodiments. To illustrate, the graph 700 includes a y-axis 702 showing peak force (hardness) in grams and an x-axis 704 showing the preparation of the comestible cell-based food product.

As shown in FIG. 7, a bar 706 corresponding to a cooked comestible cell-based food product with 100% adherent cells shows a hardness of approximately 5,020.4 grams of peak force. Additionally, bar 708 corresponding to a cooked comestible cell-based food product with 95% adherent cells and 5% dry cultured animal cell powder shows a hardness of approximately 12,313.2 grams of peak force. Also, bar 710 corresponding to a cooked comestible cell-based food product with 80% adherent cells and 20% dry cultured animal cell powder shows a hardness of approximately 23,295.1 grams of peak force. Thus, FIG. 7 illustrates how adding dry cultured animal cell powder can modify or tailor the hardness of a cooked comestible cell-based food product.

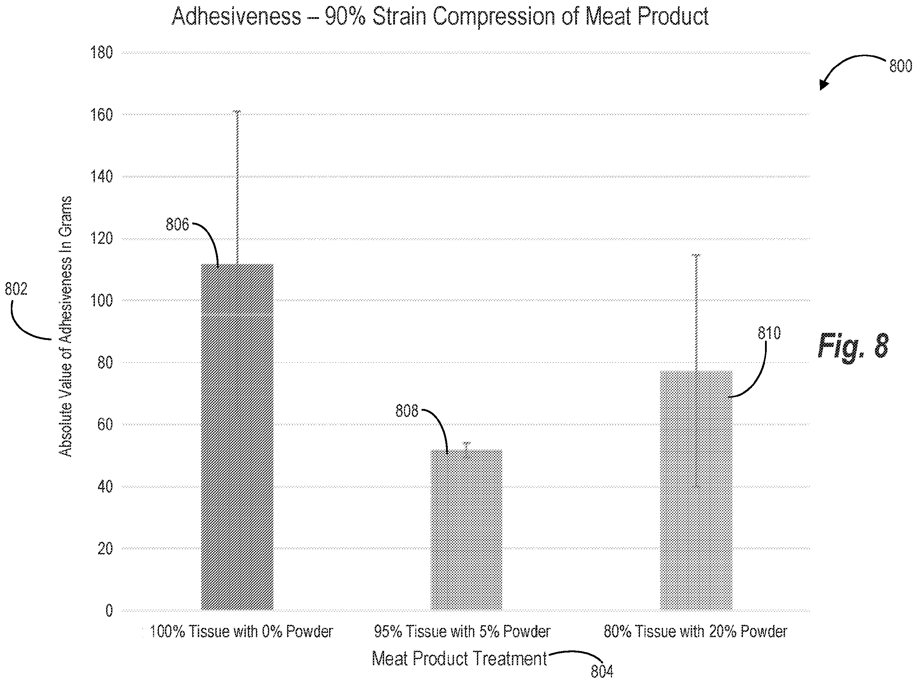

FIG. 8 illustrates adhesiveness at 90% strain compression of cooked comestible cell-based food products with various proportions of dry cultured animal cell powder in accordance with one or more embodiments. To illustrate, the graph 800 includes a y-axis 802 showing the absolute value of adhesiveness in grams and an x-axis 804 showing the preparation of the comestible cell-based food product.

As shown in FIG, 8, a bar 806 corresponding to a cooked comestible cell-based food product with 100% adherent cells shows an absolute value of adhesiveness of approximately 111.8 grams. Additionally, bar 808 corresponding to a cooked comestible cell-based food product with 95% adherent cells and 5% dry cultured animal cell powder shows an absolute value of adhesiveness of approximately 51.8 grams. Further, bar 810 corresponding to a cooked comestible cell-based food product with 80% adherent cells and 20% dry cultured animal cell powder shows an absolute value of adhesiveness of approximately 77.4 grams. Thus, FIG. 8 illustrates how adding dry cultured animal cell powder can modify or tailor the adhesiveness of a cooked comestible cell-based food product.

While the examples tested in connection with FIGS. 5-8 comprised adherent cells combined with dry cultured animal cell powder, other implementations comprise a comestible cell-based meat product produced from suspension cells and dry cultured animal cell powder or a combination of adherent cells and suspension cells together with dry cultured animal cell powder. In such implementations, the percent weight of dry cultured animal cell powder to tailor the texture (e.g., hardness and/or adhesiveness) may be higher than the values shown in connection with FIGS. 5-8. In other words, the type of cultured animal cells used in the comestible cell-based food product can influence the amount of dry cultured animal cell powder to include in a comestible cell-based food product to achieve a desired texture. Specifically, an adherent cell-based food product may require less dry cultured animal cell powder than a suspension cell-based food product to achieve a desired texture. Along similar lines, a comestible cell-based food product comprising both adherent cells and suspension cells may require less dry cultured animal cell powder than a suspension cell-based food product and more dry cultured animal cell powder than an adherent cell-based food product.

### EXAMPLE 1: Adherent and Suspension Chicken Cell Mixture with Dehydrated Chicken Powder or Similar Plant Derived Substitute, Salt, and Water

A mixture of 70% adherent cells and 30% suspension cells, comprising at least 50% by weight of the total composition, is prepared by mixing until the cells adhere to one another. The mixture is then processed and chopped into roughly ¼ inch pieces. The pieces are introduced to a salt brine and tumbled before being removed and introduced to a dehydrated animal cell powder or similar plant-based substitute or a combination of both. The dehydrated animal cell powder or similar plant substitute is added to the mixture until the powder comprises up to 20% of the total composition by weight. Salt is added until it comprises up to 3% of the total composition by weight. Sodium tripolyphosphate is added until it comprises up to 0.5% of the entire composition by weight. Stabilizers, such as pre-gel tapioca and corn starches, and carrageenan and xanthan gums, are added until they comprise up to 13% of the entire composition by weight. Additional spices and flavoring agents are added until they comprise the remainder of the entire composition by weight. The mixture is then shaped into a desired form and cooked for consumption.

### EXAMPLE 2: Adherent and Suspension Chicken Cell Mixture with Dehydrated Chicken Powder or Similar Plant Derived Substitute, Salt, Water, Phosphate, Stabilizers, Spices, and Flavoring Agents

A mixture of 70% adherent and 30% suspension cells, comprising at least 97% by weight of the entire composition, is prepared by mixing until the cells adhere to one another. The mixture is then processed and chopped into roughly ¼ inch pieces. The pieces are introduced to a salt brine and tumbled before being removed and introduced to a dehydrated animal cell powder or similar plant-based substitute or a combination of both. The dehydrated animal cell powder or similar plant substitute is added to the mixture until the powder comprises up to 1%% of the entire composition by weight. Salt is added until it comprises up to 0.5% of the entire composition by weight. Sodium tripolyphosphate is added until it comprises up to 0.5% of the entire composition by weight. Stabilizers, such as pre-gel tapioca and corn starches, and carrageenan and xanthan gums, are added until they comprise 0.5% of the entire composition by weight. Additional spices and flavoring agents are added until they comprise the remainder of the entire composition by weight.

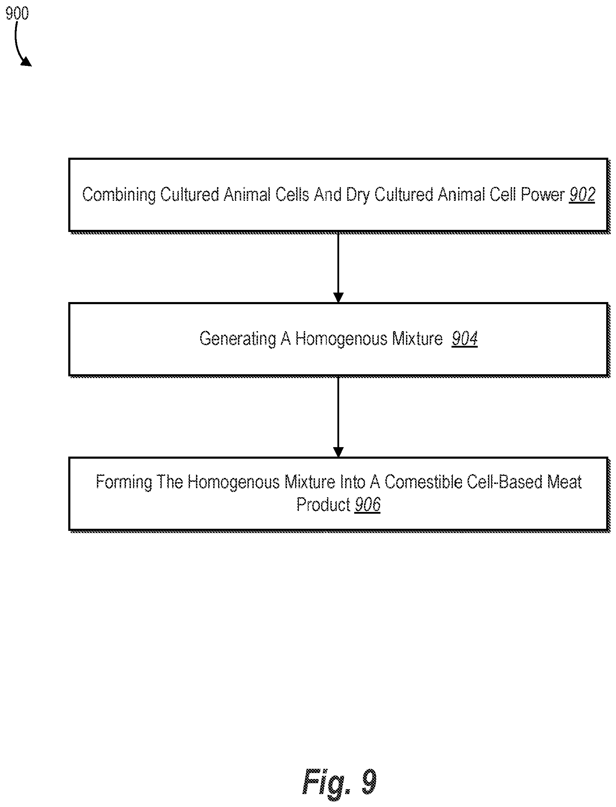

FIGS. 1-8, the corresponding text, and the examples provide several different systems, methods, techniques, components, and/or devices relating to tailoring one or more properties of a comestible cell-based food product utilizing cultured dry cell powder in accordance with one or more embodiments. In addition to the above description, one or more embodiments can also be described in terms of flowcharts including acts for accomplishing a particular result. FIGS. 9-10 illustrate such flowcharts of acts. The acts described herein may be repeated or performed in parallel with one another or in parallel with different instances of the same or similar acts.

FIG. 9 illustrates a flowchart of a series of acts 900. By way of overview, the series of acts 900 includes an act 902 of combining cultured animal cells and dry cultured animal cell powder, an act 904 of generating a homogeneous mixture from the combination of cultured animal cells and the dry cultured animal cell powder, and an act 906 of forming the homogeneous mixture into a shape of a food product.

The series of acts 900 illustrated in FIG. 9 includes the act 902 of combining cultured animal cells and dry cultured animal cell powder. In particular, the act 902 comprises combining one or more of cultured suspension cells or cultured adherent cells and dry cultured animal cell powder. In some embodiments, the act 902 comprises combining cultured animal adherent cells and the dry cultured animal cell powder. For example, act 902 can involve harvesting a cell sheet of cultured animal adherent cells from a bioreactor. Act 902 can also involve reducing a size of the cell sheet. For instance, act 902 can involve chopping the cell sheet to reduce a size of agglomerations of cultured animal adherent cells. Act 902 can further involve reducing a moisture content of the agglomerations of cultured animal adherent cells. In one or more embodiments, reducing the moisture content of the agglomerations of cultured animal adherent cells comprises vacuum drying the cultured animal adherent cells.

In one or more embodiments, act 902 involves combining cultured animal cells and dry cultured animal cell powder to form a combination comprising between 1% and 30% dry cultured animal cell powder by weight and 70% and 99% by weight cultured animal cells. In one or more embodiments, act 902 involves tailoring the amount of dry cultured animal cell powder based on the type of cultured animal cells (e.g., adherent vs. suspension vs. a combination of adherent and suspension). In still further embodiments, act 902 involves tailoring the amount of dry cultured animal cell powder based on the type of comestible cell-based food product being formed (e.g., chicken vs. ground beef vs. shrimp).

The act 902 can further involve preparing the dry cultured animal cell powder. For example, act 902 an involve growing animal cells in suspension. Act 902 can involve spray drying the cultured suspension cells to form the dry cultured animal cell powder. Alternatively, preparing the dry cultured animal cell powder can involve dehydrating a portion of agglomerations of cultured animal adherent cells and grinding them into a powder to form the dry cultured animal cell powder.

The series of acts 900 includes the act 904 of generating a homogeneous mixture from the combination of cultured animal cells and the dry cultured animal cell powder. In particular, in one or more embodiments, the act 904 comprises mixing the cultured animal cells and the dry cultured animal cell powder under a vacuum.

Optionally, act 904 involves adding at least one stabilizing binding agent to provide structure to the comestible cell-based food product during formation and cooking. For example, act 904 can involve adding one or more of polysaccharides, gums, or starches to the homogeneous mixture. Optionally, act 904 further involves adding at least one component to enhance flavor, texture, structural integrity, or nutritional value to the comestible cell-based food product. For instance, act 904 can involve adding one or more of salt, water, phosphates, plant-based protein isolates, preservatives, spices, or various vitamins and minerals to the homogeneous mixture.

FIG. 9 further includes the act 906 of forming the homogeneous mixture into a shape of a food product. For example, the act 906 comprises vacuum forming the homogeneous mixture into a shape of a chicken breast, a ground meat patty, a shrimp tail, a tube of sausage, etc.

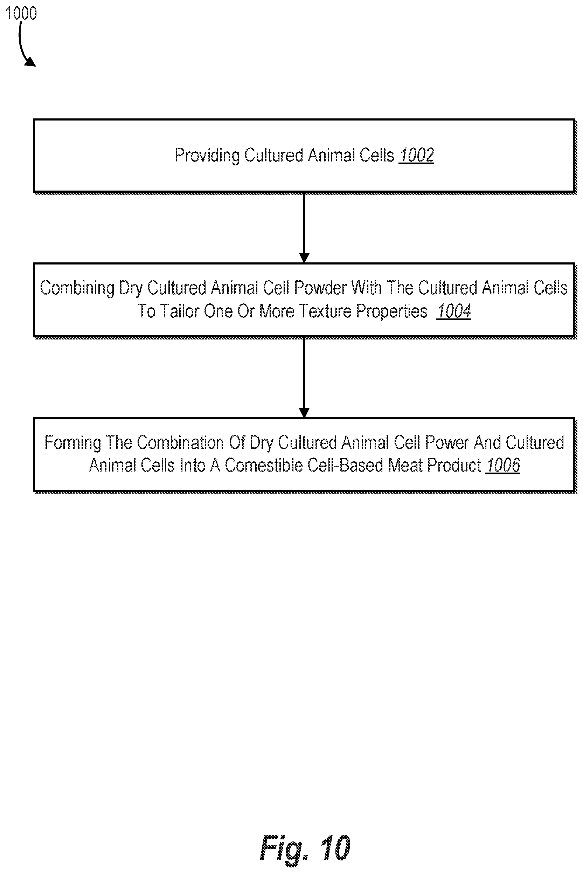

FIG. 10 illustrates a series of acts 1000. By way of overview, the series of acts 1000 includes an act 1002 of providing cultured animals cells, an act 1004 of combining dry cultured animal cell powder with the cultured animal cells to tailor one or more texture properties, and an act 1006 of forming the combination of dry cultured animal cell powder and the cultured animal cells into a shape of a meat product.

The series of acts 1000 includes the act 1002 of providing cultured animal cells . For example, act 1002 can involve growing a first set of cells and a second set of cells. In particular, the act 1002 comprises growing in suspension a first set of cells and a second set of cells. In some embodiments, the first set of cells comprises cells of a first type from at least one of myocytes, adipocytes, or fibroblasts and the second set of cells comprises cells of a second type from at least one of myocytes, adipocytes, or fibroblasts. Act 1002 can also involve seeding the first and set of cells. In particular, the act 1002 comprises seeding the first set of cells onto a substrate. In some embodiments, the act 1002 comprises seeding the first set of cells onto the substrate by circulating the first set of cells over the substrate until an outflow cell density falls below an outflow cell density threshold. In some implementations, the substrate comprises stainless steel, and the first set of cells comprises fibroblasts. Act 1002 can further involve growing the seeded first set of cells into a cellular tissue (e.g., an adherent cell sheet).

The series of acts 1000 can further involve forming dry cultured animal cell powder. For example, the series of acts 1000 can involve forming dry cultured animal cell powder from the second set of cells grown in suspension. For example, the series of acts 1000 can involve spray drying the second set of cells grown in suspension.

FIG. 10 further illustrates the act 1004 of combining dry cultured animal cell powder with the cultured animal cells to tailor one or more texture properties. For example, in one or more embodiments, act 1004 involves combining the dry cultured animal cell powder with the cultured animal cells to increase a hardness of the comestible cell-based meat product. Additionally, or alternatively, act 1004 involves combining the dry cultured animal cell powder with the cultured animal cells to increase an adhesiveness of the comestible cell-based meat product.

FIG. 10 further includes the act 1006 of forming the combination of dry cultured animal cell powder and the cultured animal cells into a shape of a meat product. For example, the act 1006 comprises vacuum forming the combination of dry cultured animal cell powder and the cultured animal cells into a shape of a chicken breast, a ground meat patty, a shrimp tail, a tube of sausage, etc.

In one or more embodiments, the series of acts 1000 involves cooking the comestible cell-based meat product. In such embodiments, the cooked comestible cell-based meat product comprises an adhesiveness having a percent difference of less than 30 percent compared to an adhesiveness of a corresponding conventional slaughtered cooked meat. For example, the cooked comestible cell-based meat product comprises an adhesiveness having a percent difference of less than 25 percent, less than 20 percent, less than 15 percent, less than 10 percent, or less than 5 percent compared to an adhesiveness of a corresponding conventional slaughtered cooked meat.

Additionally, the cooked comestible cell-based meat product can comprise a hardness having a percent difference of less than 30 percent compared to a hardness of a corresponding conventional slaughtered cooked meat. For example, the cooked comestible cell-based meat product comprises a hardness having a percent difference of less than 25 percent, less than 20 percent, less than 15 percent, less than 10 percent, or less than 5 percent compared to a hardness of a corresponding conventional slaughtered cooked meat.

In accordance with common practice, the various features illustrated in the drawings may not be drawn to scale. The illustrations presented in the present disclosure are not meant to be actual views of any particular apparatus (e.g., device, system, etc.) or method, but are merely idealized representations that are employed to describe various embodiments of the disclosure. Accordingly, the dimensions of the various features may be arbitrarily expanded or reduced for clarity. In addition, some of the drawings may be simplified for clarity. Thus, the drawings may not depict all of the components of a given apparatus (e.g., device) or all operations of a particular method.

Terms used herein and especially in the appended claims (e.g., bodies of the appended claims) are generally intended as "open" terms (e.g., the term "including" should be interpreted as "including, but not limited to," the term "having" should be interpreted as "having at least," the term "includes" should be interpreted as "includes, but is not limited to," etc.).

Additionally, if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." or "one or more of A, B, and C, etc." is used, in general such a construction is intended to include A alone, B alone, C alone, A and B together, A and C together, B and C together, or A, B, and C together, etc. For example, the use of the term "and/or" is intended to be construed in this manner.

Further, any disjunctive word or phrase presenting two or more alternative terms, whether in the description, claims, or drawings, should be understood to contemplate the possibilities of including one of the terms, either of the terms, or both terms. For example, the phrase "A or B" should be understood to include the possibilities of "A" or "B" or "A and B."

However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations.

Additionally, the use of the terms "first," "second," "third," etc., are not necessarily used herein to connote a specific order or number of elements. Generally, the terms "first," "second," "third," etc., are used to distinguish between different elements as generic identifiers. Absent a showing that the terms "first," "second," "third," etc., connote a specific order, these terms should not be understood to connote a specific order. Furthermore, absent a showing that the terms "first," "second," "third," etc., connote a specific number of elements, these terms should not be understood to connote a specific number of elements. For example, a first widget may be described as having a first side and a second widget may be described as having a second side. The use of the term "second side" with respect to the second widget may be to distinguish such side of the second widget from the "first side" of the first widget and not to connote that the second widget has two sides.

All examples and conditional language recited herein are intended for pedagogical objects to aid the reader in understanding the invention and the concepts contributed by the inventor to furthering the art and are to be construed as being without limitation to such specifically recited examples and conditions.

Indeed, the described embodiments are to be considered in all respects only as illustrative and not restrictive. For example, the methods described herein may be performed with less or more steps/acts or the steps/acts may be performed in differing orders. Additionally, the steps/acts described herein may be repeated or performed in parallel to one another or in parallel to different instances of the same or similar steps/acts. The scope of the invention is, therefore, indicated by the appended claims rather than by the foregoing description.

## Claims

1. A comestible cell-based food product comprising:
wet cultured animal cells; and
dry cultured animal cell powder;
wherein the dry cultured animal cell powder modifies a texture of the comestible cell-based food product.

2. The comestible cell-based food product of claim 1, wherein the dry cultured animal cell powder modifies the texture of the comestible cell-based food product by increasing a hardness of the comestible cell-based food product.

3. The comestible cell-based food product of claim 1, wherein the dry cultured animal cell powder modifies the texture of the comestible cell-based food product by increasing an adhesiveness of the comestible cell-based food product.

4. The comestible cell-based food product of claim 1, wherein the dry cultured animal cell powder comprises spray dried suspension cells.

5. The comestible cell-based food product of claim 1, wherein the wet cultured animal cells comprise adherent cells; and/or
wherein the wet cultured animal cells comprise suspension cells; and/or
wherein the wet cultured animal cells comprise a combination of adherent cells and suspension cells.

6. The comestible cell-based food product of claim 1, wherein comestible cell-based food product comprises between 1% and 30% dry cultured animal cell powder by weight.

7. The comestible cell-based food product of claim 6, wherein comestible cell-based food product comprises between 70% and 99% by weight wet cultured animal cells;
optionally, wherein comestible cell-based food product further comprises at least one stabilizing binding agent to provide structure to the comestible cell-based food product during formation and cooking.

8. The comestible cell-based food product of claim 1, wherein the comestible cell-based food product is a cooked product and comprises a hardness having a percent difference of less than 30 percent compared to a hardness of a corresponding conventional slaughtered meat cooked to 165 degrees Fahrenheit with no additional ingredients.

9. The comestible cell-based food product of claim 1, wherein the comestible cell-based food product is a cooked product and comprises an adhesiveness having a percent difference of less than 30 percent compared to an adhesiveness of a corresponding conventional slaughtered meat cooked to 165 degrees Fahrenheit with no additional ingredients.

10. A method of making comestible cell-based food product, the method comprising:
combining cultured animal cells and dry cultured animal cell powder;
generating a homogeneous mixture from the combination of cultured animal cells and the dry cultured animal cell powder; and
forming the homogeneous mixture into a shape of a food product.

11. The method of claim 10, further comprising preparing the dry cultured animal cell powder by spray drying cultured suspension cells.

12. The method of claim 10, wherein combining the cultured animal cells and the dry cultured animal cell powder comprises combining cultured animal adherent cells and the dry cultured animal cell powder;
optionally, further comprising:
harvesting a cell sheet of cultured animal adherent cells; and
chopping the cell sheet;
optionally, further comprising reducing a moisture content of the agglomerations of cultured animal adherent cells.

13. A method of making comestible cell-based meat product, the method comprising:
providing cultured animals cells;
combining dry cultured animal cell powder with the cultured animal cells to tailor one or more texture properties; and
forming the combination of dry cultured animal cell powder and the cultured animal cells into a shape of a meat product.

14. The method of claim 13, wherein combining the dry cultured animal cell powder with the cultured animal cells to tailor one or more texture properties comprises increasing a hardness of the comestible cell-based meat product.

15. The method of claim 13, wherein combining the dry cultured animal cell powder with the cultured animal cells to tailor one or more texture properties comprises increasing an adhesiveness of the comestible cell-based meat product.

## Patentansprüche

1. Essbares Lebensmittelprodukt auf Zellbasis, umfassend:
feucht kultivierte tierische Zellen; und
trocken kultiviertes tierisches Zellpulver;
wobei das trocken kultivierte tierische Zellpulver die Textur des essbaren Lebensmittelprodukts auf Zellbasis modifiziert.

2. Essbares Lebensmittelprodukt auf Zellbasis nach Anspruch 1, wobei das Trocken kultivierte tierische Zellpulver die Textur des essbaren Lebensmittelprodukts auf Zellbasis durch Erhöhung der Härte des essbaren Lebensmittelprodukts auf Zellbasis modifiziert.

3. Essbares Lebensmittelprodukt auf Zellbasis nach Anspruch 1, wobei das Trocken kultivierte tierische Zellpulver die Textur des essbaren Lebensmittelprodukts auf Zellbasis durch Erhöhung der Klebrigkeit des essbaren Lebensmittelprodukts auf Zellbasis modifiziert.

4. Essbares Lebensmittelprodukt auf Zellbasis nach Anspruch 1, wobei das trockene kultivierte tierische Zellpulver sprühgetrocknete Suspensionszellen umfasst.

5. Essbares Lebensmittelprodukt auf Zellbasis nach Anspruch 1, wobei die feucht kultivierten tierischen Zellen adhärente Zellen umfassen; und/oder
wobei die feucht kultivierten tierischen Zellen Suspensionszellen umfassen; und/oder
wobei die feucht kultivierten tierischen Zellen eine Kombination aus adhärenten Zellen und Suspensionszellen umfassen.

6. Essbares Lebensmittelprodukt auf Zellbasis nach Anspruch 1, wobei das essbare Lebensmittelprodukt auf Zellbasis zwischen 1 Gewichtsprozent und 30 Gewichtsprozent trocken kultiviertes tierisches Zellpulver umfasst.

7. Essbares Lebensmittelprodukt auf Zellbasis nach Anspruch 6, wobei das essbare Lebensmittelprodukt auf Zellbasis zwischen 70 und 99 Gewichtsprozent feucht kultivierte tierische Zellen umfasst;
optional, wobei das essbare Lebensmittelprodukt auf Zellbasis ferner mindestens ein stabilisierendes Bindemittel umfasst, um dem essbaren Lebensmittelprodukt auf Zellbasis während der Formung und des Kochens Struktur zu verleihen.

8. Essbares Lebensmittelprodukt auf Zellbasis nach Anspruch 1, wobei das essbare Lebensmittelprodukt auf Zellbasis ein gekochtes Produkt ist und eine Härte aufweist, die eine prozentuale Abweichung von weniger als 30 Prozent gegenüber der Härte eines entsprechenden herkömmlichen Schlachtfleisches aufweist, das ohne zusätzliche Zutaten auf 165 Grad Fahrenheit gekocht wurde.

9. Essbares Lebensmittelprodukt auf Zellbasis nach Anspruch 1, wobei das essbare Lebensmittelprodukt auf Zellbasis ein gekochtes Produkt ist und eine Klebrigkeit aufweist, die eine prozentuale Abweichung von weniger als 30 Prozent im Vergleich zur Klebrigkeit eines entsprechenden herkömmlichen Schlachtfleisches aufweist, das ohne zusätzliche Zutaten auf 165 Grad Fahrenheit gekocht wurde.

10. Verfahren zur Herstellung eines essbaren Lebensmittelprodukts auf Zellbasis, das Verfahren umfassend:
das Kombinieren von kultivierten tierischen Zellen und trockenem kultiviertem tierischem Zellpulver;
das Erzeugen einer homogenen Mischung aus der Kombination von kultivierten tierischen Zellen und dem trockenen kultivierten tierischem Zellpulver; und
das Formen der homogenen Mischung in die Form eines Lebensmittelprodukts.

11. Verfahren nach Anspruch 10, das ferner das Herstellen des trockenen kultivierten tierischen Zellpulvers durch Sprühtrocknen von kultivierten Suspensionszellen umfasst.

12. Verfahren nach Anspruch 10, wobei das Kombinieren der kultivierten tierischen Zellen und des trockenen kultivierten tierischen Zellpulvers das Kombinieren von kultivierten tierischen adhärenten Zellen und dem trockenen kultivierten tierischen Zellpulver umfasst;
optional ferner umfassend:
das Ernten einer Zellschicht aus kultivierten tierischen adhärenten Zellen; und
das Zerkleinern der Zellschicht;
optional ferner umfassend das Verringern des Feuchtigkeitsgehalts der Agglomerate aus kultivierten tierischen adhärenten Zellen.

13. Verfahren zur Herstellung eines essbaren Fleischprodukts auf Zellbasis, wobei das Verfahren umfasst:
Bereitstellen von kultivierten tierischen Zellen;
Kombinieren von trockenem kultiviertem tierischem Zellpulver mit den kultivierten tierischen Zellen, um eine oder mehrere Textureigenschaften anzupassen; und
Formen der Kombination aus trockenem kultiviertem tierischem Zellpulver und den kultivierten tierischen Zellen in die Form eines Fleischprodukts.

14. Verfahren nach Anspruch 13, wobei das Kombinieren des trockenen kultivierten tierischen Zellpulvers mit den kultivierten tierischen Zellen zum Anpassen einer oder mehrerer Textureigenschaften das Erhöhen der Härte des essbaren Fleischprodukts auf Zellbasis umfasst.

15. Verfahren nach Anspruch 13, wobei das Kombinieren des trockenen kultivierten tierischen Zellpulvers mit den kultivierten tierischen Zellen zum Anpassen einer oder mehrerer Textureigenschaften das Erhöhen der Klebrigkeit des essbaren Fleischprodukts auf Zellbasis umfasst.

## Revendications

1. Produit alimentaire comestible à base de cellules comprenant :
des cellules animales cultivées humides ; et
de la poudre de cellules animales cultivées sèches ;
dans lequel la poudre de cellules animales cultivées sèches modifie la texture du produit alimentaire comestible à base de cellules.

2. Produit alimentaire comestible à base de cellules selon la revendication 1, dans lequel la poudre de cellules animales cultivées sèches modifie la texture du produit alimentaire comestible à base de cellules en augmentant la dureté du produit alimentaire comestible à base de cellules.

3. Produit alimentaire comestible à base de cellules selon la revendication 1, dans lequel la poudre de cellules animales cultivées sèches modifie la texture du produit alimentaire comestible à base de cellules en augmentant l'adhésivité du produit alimentaire comestible à base de cellules.

4. Produit alimentaire comestible à base de cellules selon la revendication 1, dans lequel la poudre de cellules animales cultivées sèches comprend des cellules en suspension séchées par atomisation.

5. Produit alimentaire comestible à base de cellules selon la revendication 1, dans lequel les cellules animales cultivées humides comprennent des cellules adhérentes ; et/ou
dans lequel les cellules animales cultivées humides comprennent des cellules en suspension ; et/ou
dans lequel les cellules animales cultivées humides comprennent une combinaison de cellules adhérentes et de cellules en suspension.

6. Produit alimentaire comestible à base de cellules selon la revendication 1, dans lequel le produit alimentaire comestible à base de cellules comprend entre 1 % et 30 % en poids de poudre de cellules animales cultivées sèches.

7. Produit alimentaire comestible à base de cellules selon la revendication 6, dans lequel le produit alimentaire comestible à base de cellules comprend entre 70 % et 99 % en poids de cellules animales cultivées humides ;
facultativement, dans lequel le produit alimentaire comestible à base de cellules comprend en outre au moins un agent liant stabilisant pour fournir une structure au produit alimentaire comestible à base de cellules pendant la formation et la cuisson.

8. Produit alimentaire comestible à base de cellules selon la revendication 1, dans lequel le produit alimentaire comestible à base de cellules est un produit cuit et comprend une dureté présentant une différence en pourcentage inférieure à 30 % par rapport à la dureté d'une viande abattue conventionnelle correspondante cuite à 165 degrés Fahrenheit sans ingrédients supplémentaires.

9. Produit alimentaire comestible à base de cellules selon la revendication 1, dans lequel le produit alimentaire comestible à base de cellules est un produit cuit et comprend une adhérence présentant une différence en pourcentage inférieure à 30 % par rapport à l'adhérence d'une viande abattue conventionnelle correspondante cuite à 165 degrés Fahrenheit sans ingrédients supplémentaires.

10. Procédé de fabrication d'un produit alimentaire comestible à base de cellules, le procédé comprenant :
la combinaison de cellules animales cultivées et de poudre de cellules animales cultivées sèches ;
la génération d'un mélange homogène à partir de la combinaison de cellules animales cultivées et de la poudre de cellules animales cultivées sèches ; et
la mise en forme du mélange homogène en un produit alimentaire.

11. Procédé selon la revendication 10, comprenant en outre la préparation de la poudre de cellules animales cultivées sèches par séchage par atomisation de cellules en suspension cultivées.

12. Procédé selon la revendication 10, dans lequel la combinaison des cellules animales cultivées et de la poudre de cellules animales cultivées sèches comprend la combinaison de cellules animales adhérentes cultivées et de la poudre de cellules animales cultivées sèches ;
comprenant en outre, de manière facultative :
la récolte d'une feuille cellulaire de cellules animales adhérentes cultivées ; et
le hachage de la feuille cellulaire ;
comprenant en outre, de manière facultative, la réduction de la teneur en humidité des agglomérats de cellules animales adhérentes cultivées.

13. Procédé de fabrication d'un produit carné comestible à base de cellules, le procédé comprenant :
la fourniture de cellules animales cultivées ;
la combinaison de la poudre de cellules animales cultivées sèches avec les cellules animales cultivées pour adapter une ou plusieurs propriétés de texture ; et
la mise en forme de la combinaison de la poudre de cellules animales cultivées sèches et des cellules animales cultivées en un produit carné.

14. Procédé selon la revendication 13, dans lequel la combinaison de la poudre de cellules animales cultivées sèches avec les cellules animales cultivées pour adapter une ou plusieurs propriétés de texture comprend l'augmentation de la dureté du produit carné comestible à base de cellules.

15. Procédé selon la revendication 13, dans lequel la combinaison de la poudre de cellules animales cultivées sèches avec les cellules animales cultivées pour adapter une ou plusieurs propriétés de texture comprend l'augmentation de l'adhésivité du produit carné comestible à base de cellules.
